# EUROPÄISCHE PATENTSCHRIFT

(11) **EP 1 802 578 B1**
(45) Veröffentlichungstag und Bekanntmachung des Hinweises auf die Patenterteilung: **23.04.2008**
(21) Anmeldenummer: 05788702.8
(22) Anmeldetag: 05.10.2005
(51) Int. Cl.: C07D 213/76, C07D 213/69, C07D 401/12, A61K 31/4412, A61K 31/4439, A61K 31/444, A61P 9/10, A61P 25/28

(54) **3,6-SUBSTITUIERTE 5-ARYLAMINO-1H-PYIDIN-2-ON DERIVATIVE UND VERWANDTE VERBINDUNGEN ALS POLY(ADP-RIBOSE)POLYMERASE (PARP) INHIBITOREN ZUR BEHANDLUNG VON DURCH NEKOSE ODER APOPTOSE VERURSACHTEN GEWEBESCHÄDEN ODER ERKANKUNGEN**
3,6-SUBSTITUTED 5-ARYLAMINO-1H-PYIDINE-2-ONE DERIVATIVES AND RELATED COMPOUNDS AS POLY(ADP-RIBOSE)POLYMERASE (PARP) INHIBITORS IN THE TREATMENT OF TISSUE DAMAGE OR DISEASE CAUSED BY NECROSIS OR APOPTOSIS
DERIVES DE 5-ARYLAMINO-1H-PYRIDINE-2-ON SUBSTITUES SUR 3,6 ET COMPOSES SIMILAIRES UTILISES EN TANT QU'INHIBITEURS DE LA POLYMERASE POLY (ADP-RIBOSE) DESTINES AU TRAITEMENT DE MALADIES OU D'ENDOMMAGEMENTS DES TISSUS PROVOQUES PAR UNE NECROSE OU UNE APOPTOSE

(30) Priorität: 15.10.2004 DE 102004050196
(43) Veröffentlichungstag der Anmeldung: 04.07.2007
(73) Patentinhaber: Sanofi-Aventis Deutschland GmbH, 65929 Frankfurt am Main (DE)
(72) Erfinder: PEUKERT, Stefan, Arlington, MA 02476 (US); GUESSREGEN, Stefan, 65205 Wiesbaden (DE); HOFMEISTER, Armin, 55278 Dexheim (DE); SCHREUDER, Hermann, 65719 Hofheim-Lorsbach (DE); SCHWAHN, Uwe c/o Sanofi-Aventis Deutschland GmbH, 65926 Frankfurt am Main (DE)
(86) Internationale Anmeldenummer: PCT/EP2005/010697
(87) Internationale Veröffentlichungsnummer: WO 2006/042638

(56) Entgegenhaltungen:
- EP-A- 0 109 628
- WO-A-93/07137
- WO-A-03/063874

## Beschreibung

Die Erfindung betrifft Verbindungen der allgemeinen Formel I, 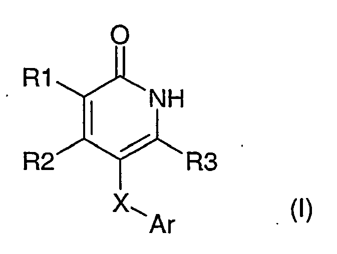 wobei die Definitionen der Substituenten R1, R2, R3, Ar und X im nachfolgenden Text aufgeführt sind, sowie deren physiologisch verträgliche Salze, Verfahren zur Herstellung dieser Verbindungen und deren Verwendung als Arzneimittel.
Diese Verbindungen sind Inhibitoren von Poly(ADP-ribose)polymerase (PARP).

Poly(adenosin 5'-diphosphatribose)polymerase [Poly(ADP-ribose)polymerase, PARP], die auch als Poly(ADP-ribose)synthetase (PARS) bekannt ist, ist ein Chromatingebundenes nukleäres Enzym von eukaryoten Zellen, das mit ungefähr 2 x 10⁵ Molekülen pro Kern vorhanden ist. PARP spielt gemäß jüngsten Forschungsergebnissen in der Pathogenese verschiedener Erkrankungen eine Rolle und kann somit die Inhibition der PARP-Enzymaktivität den Verlauf von Erkrankungen in präklinischen Tiermodellen positiv beeinflussen (Cristina Cosi, Expert Opin. Ther. Patents, 2002, 12, 1047-1071 und L. Virag und C. Szabo, Pharmacol. Rev., 2002, 54, 1-54). Die Poly(ADP-ribose)polymerase kommt in allen eukaryontischen Organismen mit Ausnahme der Hefe vor und ist Teil des Genomüberwachungsnetzwerkes zum Schutz der Erbinformation gegen genotoxische Einflüsse. DNS-Schädigungen induzieren die enzymatische Aktivität der Poly(ADP-ribose)polymerase, was unter physiologischen Bedingungen zur Reparatur der vom Enzym erkannten Fehler der DNS führt. Unter pathologischen Umständen kann es jedoch zu einer starken Aktivierung der Poly(ADP-ribose)polymerase durch radikale Sauerstoffspezies kommen - wie bei der Ischämie, Hypoxie, Reperfusion oder bei Entzündungsprozessen der Fall - wodurch das Enzym große Mengen seines Substrats NAD verbraucht. Diese Verarmung an NAD ist mit ein Grund für den im betroffenen Gewebe zu beobachtenden Untergang von Zellen (die sogenannte Energiekrisen-Hypothese). Der therapeutische Nutzen von PARP-Inhibitoren liegt in der Verhinderung bzw. Minderung dieser NAD-Verarmung im Gewebe. Außer der hier beschriebenen Rolle in der Signalübertragung von oxidativem Stress in Zellen hin zur NAD-Verarmung werden in der aktuellen Literatur weitere zelluläre Funktionen der PARP diskutiert, die ebenfalls eine Rolle für den molekularen Wirkmechanismus von PARP-Inhibitoren unter pathologischen Umständen spielen könnten (A. Chiarugi, Trends Pharmacol. Sci., 2002, 23, 122-129). Unabhängig von dieser offenen Diskussion um den molekularen Wirkmechanismus konnte in mehreren präklinischen Tiermodellen die therapeutische Effektivität von verschiedenen PARP-Inhibitoren gezeigt werden: So zum Beispiel für den akuten Myokardinfarkt, akutes Nierenversagen, cerebrale Ischämie (Schlaganfall), neurodegenerative Erkrankungen (z.B. ein Modell der Parkinsonschen Krankheit), Diabetes, Xenobiotika-induzierte Hepatotoxizität, Arthritis, Schocklunge, septischen Schock und als Sensitivierer in der Chemotherapie neoplastischer Erkrankungen (Zusammengefasst in L. Virag und C. Szabo, Pharmacol. Rev., 2002, 54, 1-54).

Im Einzelnen konnte gezeigt werden, dass PARP-Inhibitoren nicht nur im akuten Herzinfarkt morphologische und funktionelle Verbesserungen bewirken (J. Bowes et al., Eur. J. Pharmacol., 1998, 359, 143-150; L. Liaudet et al., Br. J. Pharmacol., 2001, 133,1424-1430; N. Wayman et al., Eur. J Pharmacol., 2001, 430, 93-100) sondern auch bei der chronischen Herzinsuffizienz sind unter PARP-Inhibitorbehandlung signifikant bessere Herzleistungen gemessen worden (P. Pacher, J. Am. Coll. Cardiol., 2002, 40, 1006-1016). Die Minderdurchblutung, wie sie im infarzierten Herzen Einbußen in der Leistung des Organs durch den Untergang von Zellen bewirkt, steht auch beim Schlaganfall am Anfang der Ereigniskette, die zum Verlusten oder Komplettausfall einzelner Organbereiche und damit -funktionen führt. Demzufolge konnte die Wirksamkeit von PARP-Inhibitoren - neben der genetischen Ablation des PARP-1 Gens (M.J.L. Eliasson et al., Nat. Med., 1997, 10, 1089-1095) - auch in Modellen der cerebralen Ischämie (K. Takahashi et al., L. Cereb. Blood Flow Metab., 1997,11, 1137-1142), der MPTP-induzierten Neurotoxizität (C.Cosi et al., Brain Res., 1996, 729, 264-269) und neuronaler Erregungstoxizität (A.S. Mandir et al., J. Neurosci., 2000, 21, 8005-8011) gezeigt werden. Ein weiterer im Zusammenhang von Herz-Kreislauf-Erkrankungen sehr wichtiger Befund ist die Wirksamkeit von PARP-Inhibition in der ischämisch geschädigten Niere, wo ebenfalls Verbesserungen der Filtrationsleistung des Organs bei PARP-Inhibitor-behandelten im Vergleich zu Plazebo-behandelten Tieren festgestellt werden konnten (D.R. Martin et al., Am. J. Physiol. Regulatory Integrative Comp. Physiol., 2000, 279, R1834-R1840). Im Gegensatz zu den akuten ischämischen Insulten der oben aufgeführten Erkrankungen kommt es in diversen Pathologien zur chronischen PARP-Aktivierung, wie z.B. im Diabetes. Sowohl in präklinischen Modellen das Typ-I-Diabetes (W.L. Suarez-Pinzon et al., Diabetes, 2003, 52, 1683-1688) als auch in solchen des Typ-II-Diabetes konnte die Wirksamkeit von PARP-Inhibitoren nachgewiesen werden (F.G. Soriano et al., Nat. Med., 2001, 7, 108-113; F.G. Soriano et al., Circulation, 2001, 89, 684-691). Die positive Wirkung von PARP-Inhibitoren im Typ-I-Diabetes ist auf deren antiinflammatorische Eigenschaften zurückzuführen, die auch in weiteren präklinischen Modellen, wie der chronischen Colitis (H.B. Jijon et al., Am. J. Physiol. Gastrointest. Liver Physiol., 2000, 279,G641-G651), der Kollagen-induzierten Arthritis (H. Kröger et al., Inflammation, 1996, 20, 203-215) und im septischen Schock (B. Zingarelli et al., Shock, 1996, 5, 258-264) gezeigt werden konnten. Daneben wirken PARP-Inhibitoren sensitivierend auf Tumoren in der Chemotherapie an Mäusen (L. Tentori et al., Blood, 2002, 99, 2241-2244).

Aus der Literatur ist bereits bekannt (beispielsweise C. Cosi, Expert Opin. Ther. patents, 2002, 12, 1047-1071; Southan et. al., Current Medicinal Chemistry, 2003,10, 321 - 340), dass viele unterschiedliche Klassen von chemischen Verbindungen als PARP-Inhibitoren verwendet werden können, wie beispielsweise Derivate von Indolen, Benzimidazolen, Isochinolinolen oder Chinazolinonen. Viele der bereits bekannten PARP-Inhibitoren sind Derivate eines bi- oder polycyclischen Grundgerüstes. Pyridonderivate und deren möglichen Verwendung als pharmazeutsch aktive Substanzen sind bekannt. Die Verwendung von Pyridonderivaten als PARP-Inhibitoren ist allerdings noch nicht beschrieben. Die in der Literatur beschriebenen Pyridonderivate weisen im Vergleich zu den erfindungsgemäßen Verbindungen der Formel I unterschiedliche Substitutionsmuster auf.

US 4,431,651 beschreibt 2-Pyridonderivate als Kardiotonika, die an der 5-Position einen substituierten Phenyl- oder Pyridinylrest tragen.

US 4,699,914 beschreibt 2-Pyridonderivate zur Behandlung einer Stauungsherzinsuffizienz, die an der 5-Position eine Imidazolylthienyl- oder eine jmidiazoiyiphenyl-Gruppe tragen.

EP-A 489327 beschreibt Chromanderivate, die eine Wirkung auf das cardiovaskuläre System zeigen und mit einem 2-Pyridon-aminorest substituiert sein können.

WO93/07137 beschreibt Pyridinolderivate als Proteinkinaseagonisten, die an der 3-Position verschiedenartige Substituenten tragen.

WO95/00511 beschreibt Naphthyridin- und Pyridopyrazin-Derivate mit antirheumatischen Eigenschaften, die mit einem 2-Pyridon-aminorest substituiert sein können.

WO95/13272 beschreibt Chromanderivat zur Behandlung von kardiovaskulären Erkrankungen, die mit einem Dihydroxopyridylrest substituiert sein können.

WO01/02400 beschreibt Phenyl-, Pyridinyl und Pyrimidinyl-Derivate, die neben einer Halogen- und einer Aminogruppe einen unsubstituierten bzw. einfach substituierten 2-Pyridon-amino-Rest tragen und als Zwischenprodukte zur Herstellung von kondensierten Imidazolderivaten dienen, die als Adenosin A2 Rezeptorantagonisten verwendet werden können.

WO01/25220 und US 2004/0116388 beschreiben Triazinderivate als Kinaseinhibitoren, die einen 2-Pyridonaminorest tragen können.

Da Krankheiten wie Myokardinfarkt, die durch die Inhibierung von PARP behandelt werden können, eine ernstzunehmende Gefahr für die Gesundheit von Menschen und anderen Säugern darstellt, besteht ein großer Bedarf nach neuen Arzneimitteln, die über ein vorteilhaftes therapeutisches Profil zur Behandlung von solchen Krankheiten verfügen. Der vorliegenden Erfindung liegt deshalb die Aufgabe zugrunde, neue Verbindungen bereitzustellen, die einen inhibitorischen Effekt auf PARP haben.

Die vorliegende Erfindung betrifft substituierte 2-Pyridon-Derivate der Formel I 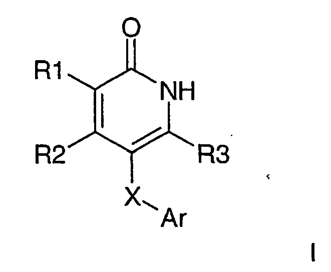 worin bedeuten:
- R1 und R3: unabhängig voneinander Fluor, Methoxy, -OCF₃, C₂-C₃-Alkenyl oder C₁-C₄-Alkyl, das gegebenenfalls mit Chlor, Methoxy oder einem, zwei oder drei Fluoratomen substituiert ist;
- R2: Wasserstoff, Fluor, Methoxy, -OCF₃, C₂-C₃-Alkenyl oder C₁-C₄-Alkyl, das gegebenenfalls mit Chlor, Methoxy oder einem, zwei oder drei Fluoratomen substituiert ist;
- X: O, S, NH oder N(C₁-C₃-Alkyl);
- Ar: unsubstituiertes oder zumindest einfach substituiertes Aryl oder Heteroaryl, wobei die Substituenten ausgewählt sind aus der Gruppe bestehend aus: Fluor, Chlor, Brom, -CF₃, -OCF₃, -NO₂, -CN, -C(O)(C₁-C₆-Alkyl), NH₂, -NHC(O)(C₁-C₆-Alkyl), Hydroxy, Oxo, C₁-C₆-Alkyl, C₁-C₆-Alkoxy, -NH(C₁-C₆-Alkyl), -N(C₁-C₆-Alkyl)₂, -SO₂(C₁-C₆-Alkyl), Heterocyclyl, Heteroaryl, Aryl, -O-Aryl, -O-Heteroaryl, -CH₂-NR4R5, -SO₂NR4R5, und -C(O)NR4R5,
wobei der Substituent C₁-C₆-Alkyl mit C₁-C₆-Alkoxy, Fluor, Chlor, Trifluormethyl, Trifluormethoxy, Aryl, Heteroaryl, Heterocyclyl oder OH zumindest einfach substituiert sein kann,
und die Substituenten Aryl, Heteroaryl und Heterocyclyl mit C₁-C₆-Alkyl, C₁-C₆-Alkoxy, Fluor, Chlor, Trifluormethyl, Trifluormethoxy oder OH zumindest einfach substituiert sein können;
- Aryl: 5 bis 10-gliedriger, aromatischer Mono- oder Bicyclus;
- Heteroaryl: 5 bis 10-gliedriger, aromatischer, mono- oder bicyclischer Heterocyclus, der ein oder mehrere Heteroatome ausgewählt aus N, O und S enthält;
- Heterocyclyl: 5 bis 10-gliedriger, nicht-aromatischer, mono- oder bicyclischer Heterocyclus, der ein oder mehrere Heteroatome ausgewählt aus N, O und S enthält;
- R4 und R5: unabhängig voneinander ausgewählt aus der Gruppe bestehend aus: Wasserstoff; unsubstituiertes oder zumindest monosubstituiertes C₁-C₁₀-Alkyl, C₂-C₆-Alkenyl, Phenyl, Indanyl, Heterocyclyl und Heteroaryl,
wobei die Substituenten ausgewählt sind aus der Gruppe bestehend aus: Phenyl, Heteroaryl, Heterocyclyl, -O-Phenyl, Fluor, -CN, -C(O)NH₂, -C(O)(C₁-C₃-Alkyl), -C(O)-Phenyl, -N(C₁-C₃-Alkyl)₂, -NH(C₁-C₃-Alkyl), -NH₂, -NH-Heteroaryl, -NH-C(O)-Heteroaryl, C₁-C₆-Alkyl, C₁-C₃-Alkoxy und Hydroxy,
und die Phenyl-, Heterocyclyl- und Heteroaryl-Fragmente dieser Substituenten können wiederum mit Fluor, Chlor, Brom, Oxo, -CF₃, -OCF₃, -NO₂, -CN, Phenyl, Pyridinyl, -NHC(O)(C₁-C₃-Alkyl), -COOH, Hydroxy, C₁-C₃-Alkyl, C₁-C₃-Alkoxy, -SO_{2NH2}, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂, zumindest monosubstituiert sein; oder
- R4 und R5: bilden zusammen mit dem Stickstoffatom, an das sie gebunden sind, unsubstituiertes oder zumindest monosubstituiertes Heterocyclyl,
wobei die Substituenten ausgewählt sind aus der Gruppe bestehend aus: Phenyl, Heteroaryl, Heterocyclyl, Oxo, Fluor, Chlor, -C(O)(C₁-C₃-Alkyl), -C(O)-Phenyl und Hydroxy,
und die Phenyl-, Heterocyclyl- und Heteroaryl-Fragmente dieser Substituenten können wiederum mit Fluor oder C₁-C₃-Alkyl zumindest monosubstituiert sein;
oder ein physiologisch verträgliches Salz davon;
mit der Maßgabe, dass Ar nicht Triazinyl oder Chromanyl bedeutet,
und wenn X NH oder N(C₁-C₃-Alkyl) ist Ar nicht Pyridopyrazinyl oder Naphthyridinyl bedeutet.

Sofern in den Verbindungen der Formel (I) Gruppen, Fragmente, Reste oder Substituenten wie beispielsweise Aryl, Heteroaryl, Alkyl, Alkoxy usw. mehr als einmal vorhanden sind, haben sie alle unabhängig voneinander die oben aufgeführten Bedeutungen und können somit in jedem (Einzel)-Fall entweder eine identische oder eine voneinander unabhängige Bedeutung haben. Die nachfolgenden Ausführungen gelten für (beispielsweise) Aryl sowie jeden beliebigen anderen Rest unabhängig von dessen Bezeichnung als Arylgruppe, -substituent, -fragment oder -rest. Ein weiteres Beispiel ist die -N(C₁-C₃-alkyl)₂ Gruppe, in der die beiden Alkylsubstituenten entweder identisch oder unterschiedlich sein können (beispielsweise zweimal Ethyl oder einmal Propyl und einmal Methyl).

Sofern in den vorstehenden Definitionen für Verbindungen der Formel (I) ein Substituent, beispielsweise Aryl, unsubstituiert oder zumindest monosubstituiert mit einer Gruppe von weiteren Substituenten, beispielsweise C₁-C₆-Alkyl, C₁-C₆-Alkoxy, Halogen usw., sein kann, so gilt für diejenigen Fälle, in denen eine Mehrfachsubstitution von Aryl vorliegt, dass die Auswahl aus der Reihe von weiteren Substituenten unabhängig voneinander erfolgt. Somit sind beispielsweise bei einer Zweifachsubstitution von Aryl sämtliche Kombinationen der weiteren Substituenten mit umfasst. Aryl kann somit beispielsweise zweifach-substituiert mit Ethyl sein, Aryl kann jeweils monosubstituiert mit Methyl und Ethoxy sein, Aryl kann jeweils monosubstituiert mit Ethyl und Fluor sein, Aryl kann zweifach-substituiert mit Methoxy sein, usw..

Alkylreste können entweder linear oder verzweigt, acyclisch oder cyclisch sein. Dies trifft auch zu, sofern sie Teil einer anderen Gruppe wie beispielsweise Alkoxygruppen (C₁-C₆-Alkyl-O-), Alkoxycarbonylgruppen oder Aminogruppen sind oder wenn sie substituiert sind.

Beispiele für Alkylgruppen sind:Methyl, Ethyl, Propyl, Butyl, Pentyl, Hexyl, Heptyl, Octyl, Nonyl oder Decyl. Dabei sind sowohl die n-Isomere dieser Reste als auch Isopropyl, Isobutyl, Isopentyl, sec-Butyl, tert-Butyl, Neopentyl, 3,3-Dimethylbutyl usw. mit umfasst. Sofern nicht anders beschrieben, beinhaltet der Begriff Alkyl darüber hinaus auch Alkylreste, die unsubstituiert oder gegebenenfalls mit einem oder mehreren weiteren Resten substituiert sind, beispielsweise 1, 2, 3 oder 4 identische oder unterschiedliche Reste, wie beispielsweise Aryl, Heteroaryl, Alkoxy oder Halogen. Dabei können die zusätzlichen Substituenten in jeder beliebigen Position des Alkylrestes auftreten. Weiterhin umfasst der Begriff Alkyl auch Cycloalkyl sowie Cycloalkyl-alkyl- (Alkyl, das wiederum mit Cycloalkyl substituiert ist), wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt. Beispiele für Alkenylgruppen sind: Vinyl, 1-Propenyl, 2-Propenyl (Allyl), 2-Butenyl, 2-Methyl-2-propenyl oder 3-Methyl-2-butenyl. Der Begriff Alkenyl umfasst hier ausdrücklich auch Cycloalkenylreste sowie Cycloalkenyl-Alkylreste (Alkyl, das mit Cycloalkenyl substituiert ist), die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen. Die Alkenylreste können unsubstituiert oder gegebenenfalls mit einem oder mehreren weiteren Resten substituiert sein, wie vorstehend bei den Alkylresten beispielhaft aufgeführt.

Sofern nicht anders ausgeführt, können die vorgenannten Aryl-, Heteroaryl- und Heterocyclylreste sowohl unsubstituiert als auch einen oder mehrere, beispielsweise 1, 2, 3 oder 4 weitere, der vorgenannten Substituenten in jeder beliebigen Position aufweisen. Beispielsweise kann in monosubstituierten Phenylresten der Substituent in der Position 2, 3 oder 4, in disubstituierten Phenylresten können die Substituenten in der 2,3-Position, 2,4-Position, 2,5-Position, 2,6-Position, 3,4-Position oder 3,5-Position sein. In dreifach substituierten Phenylresten können die Substituenten in der 2,3,4-Position, 2,3,5-Position, 2,3,6-Position, 2,4,5-Position, 2,4,6-Position oder 3,4,5-Position sein. In vierfach substituierten Phenylresten können die Substituenten in der 2,3,4,5-Position, der 2,3,4,6-Position oder in der 2,3,5,6-Position sein.

Die vorgenannten beziehungsweise die nachfolgenden Definitionen, die sich auf einwertige Reste beziehen, gelten genauso für zweiwertige Reste wie Phenylen, Naphthylen oder Heteroarylen. Diese zweiwertigen Reste (Fragmente) können mit dem benachbarten Gruppen für jedes beliebige Ring-Kohlenstoffatom verknüpft sein. Im Falle von Phenylenresten kann dies in der 1,2-Position (Ortho-Phenylen), 1,3-Position (meta-Phenylen) oder 1,4-Position (Para-Phenylen) sein. Im Falle eines 5-gliedrigen Aromaten, der ein Heteroatom enthält, wie beispielsweise Thiophen oder Furan können die beiden freien Bindungen in der 2,3-Position, 2,4-Position, 2,5-Position oder der 3,4-Position sein. Ein zweiwertiger Rest, der von einem 6-gliedrigen Aromaten mit einem Heteroatom abgeleitet ist, wie beispielsweise Pyridin, kann ein 2,3-, 2,4-, 2,5-, 2,6-, 3,4- oder 3,5-Pyridindiylrest sein. Im Falle von unsymmetrischen zweiwertigen Resten beinhaltet die vorliegende Erfindung auch alle Positionsisomere, d.h. im Falle von beispielsweise einem 2,3-Pyridindiylrest ist die Verbindung, in der die eine benachbarte Gruppe sich in der Position 2 und die andere benachbarte Gruppe in der Position 3 sich befindet, genauso wie die Verbindung, in der die eine benachbarte Gruppe in der Position 3 und die andere benachbarte Gruppe sich in der Position 2 befindet, mit umfasst.

Soweit nicht anders aufgeführt, sind Heteroarylreste, Heteroarylenreste, Heterocyclylreste und Heterocyclylenreste sowie Ringe,-die durch zwei an Stickstoff gebundene Gruppen gebildet werden, vorzugsweise abgeleitet von vollständig gesättigten, teilweise oder ganz ungesättigten Heterocyclen (d.h. Heterocycloalkane, Heterocycloalkene, Heteroaromaten), die 1, 2, 3 oder 4 Heteroatome enthalten, die sowohl unterschiedlich als auch gleich sein können. Vorzugsweise sind sie von Heterocyclen abgeleitet, die 1, 2 oder 3, besonders bevorzugt 1 oder 2, Heteroatome enthalten, die gleich oder unterschiedlich sein können. So lange nicht anders aufgeführt, sind die Heterocyclen mono- oder polycyclisch, zum Beispiel monocyclisch, bicyclisch oder tricyclisch. Vorzugsweise sind sie monocyclisch oder bicyclisch. Vorzugsweise sind es 5-gliedrige, 6-gliedrige oder 7-gliedrige Ringe, besonders bevorzugt 5-gliedriger oder 6-gliedrige Ringe. Im Fall von polycyclischen Heterocyclen mit 2 und mehr Heteroatomen können diese alle im gleichen Ring vorkommen oder auf mehrere Ringe verteilt sein.

Als Heteroaryl werden in der vorliegenden Erfindung Reste bezeichnet, die von monocyclischen oder bicyclischen, aromatischen Heterocyclen abgeleitet sind. Beispiele für Heteroaryl sind: Pyrrolyl, Furanyl (=Furyl), Thiophenyl (=Thienyl), Imidazolyl, Pyrazolyl, 1,2,3-Triazolyl, 1,2,4-Triazolyl, 1,3-Oxazolyl (=Oxazolyl), 1,2-Oxazolyl (=lsoxazolyl), Oxadiazolyl, 1,3-Thiazolyl (=Thiazolyl), 1,2-Thiazolyl (=lsothiazolyl), Tetrazolyl, Pyridinyl (=Pyridyl) Pyridazinyl, Pyrimidinyl, Pyrazinyl, 1,2,3-Triazinyl, 1,2,4-Triazinyl, 1,3,5-Triazinyl, 1,2,4,5-Tetrazinyl, Indazolyl, Indolyl, Benzothiophenyl, Benzofuranyl, Benzothiazolyl, Benzimidazolyl, Chinolinyl, Isochinolinyl, Chinazolinyl, Chinoxalinyl, Phthalazinyl, Thienothiophenyl, 1,8-Naphthyridinyl, andere Naphthyridinyle, Pteridinyl oder Thiazolo[3,2-b][1,2,4]-tiazolyl. Sofern es sich nicht um monocyclische Systeme handelt, ist bei jedem der vorgenannten Heteroaryle für den zweiten Ring auch die gesättigte Form (Perhydroform) oder die teilweise ungesättigte Form (beispielsweise die Dihydroform oder Tetrahydroform) oder die maximal ungesättigte (nicht-aromatische) Form mit umfasst, sofern die jeweiligen Formen bekannt und stabil sind. Die Bezeichnung Heteroaryl umfasst somit in der vorliegenden Erfindung beispielsweise auch bicyclische Reste, in denen sowohl beide Ringe aromatisch sind als auch bicyclische Reste, in denen nur ein Ring aromatisch ist. Solche Beispiele für Heteroaryl sind: 3H-Indolinyl, 2(1 H)-Chinolinonyl, 4-Oxo-1,4-dihydrochinolinyl, 2H-1-Oxoisochinolyl, 1,2-Dihydrochinolinyl, 3,4-Dihydrochinolinyl, 1,2-Dihydroisochinolinyl, 3,4-Dihydroisochinolinyl, Chromonyl, Chromanyl, 1,3-Benzodioxolyl, Oxindolyl, 1,2,3,4-Tetrahydroisochinolinyl, 1,2,3,4- Tetrahydrochinolinyl, 5,6-Dihydrochinolyl, 5,6-Dihydroisochinolyl, 5,6,7,8-Tetrahydrochinolinyl oder 5,6,7,8-Tetrahydroisochinolyl.

Als Heterocyclyl werden in der vorliegenden Erfindung Reste bezeichnet, die von monocyclischen oder bicyclischen, nicht-aromatischen Heterocyclen abgeleitet sind. Unter nicht-aromatischen Heterocyclen werden nachfolgend insbesondere Heterocycloalkane (vollständig gesättigte Heterocyclen) sowie Heterocycloalkene (teilweise ungesättigte Heterocyclen) verstanden. Im Fall der Heterocycloalkene werden auch Verbindungen mit zwei oder mehreren Doppelbindungen mit umfasst, die gegebenenfalls auch miteinander konjugiert sein können. Beispiele für Heterocyclyl sind: Pyrrolidinyl, Piperidinyl, Piperazinyl, Imidazolidinyl, Pyrazolidinyl, Isothiazolidinyl, Thiazolidinyl, Isoxazolidinyl, Oxazolidinyl, Tetrahydrofuranyl, Tetrahydrothiophenyl, 1,3-Dioxolanyl, 1,4-Dioxinyl, Pyranyl, Thiopyranyl, Tetrahydro-1,2-Oxazinyl, Tetrahydro-1,3-Oxazinyl, Morpholinyl, Thiomorpholinyl, 1,2-Thiazinyl, 1,3-Thiazinyl, 1,4-Thiazinyl, Azepinyl, 1,2-Diazepinyl, 1,3-Diazepinyl, 1,4-Diazepinyl, 1,3-Oxazepinyl, 1,3-Thiazepinyl, Azepanyl, 2-Oxo-azepanyl, 1,2,3,4- Tetrahydropyridinyl, 1,2-Dihydropyridinyl, 1,4-Dihydropyridinyl, 1,2,3,6-Tetrahydropyridinyl, 4(3H)-pyrimidonyl, 1,4,5,6-Tetrahydropyrimidinyl, 2-Pyrrolinyl, 3-Pyrrolinyl, 2-Imidazolinyl, 2-Pyrazolinyl, 3,4-Dihydro-2H-pyranyl, Dihydrofuranyl, 7-Oxabicyclo[2.2.1]heptenyl, Dihydrothiophenyl und Dihydrothiopyranyl. Der Grad der Sättigung von heterocyclischen Gruppen ist in der jeweiligen Definition angezeigt.

Substituenten, die von diesen Heterocyclen abgeleitet sind, können über jedes geeignete Kohlenstoffatom verknüpft sowie mit weiteren Substituenten versehen sein. Reste, die von stickstoffhaltigen Heterocyclen abgeleitet sind, können am entsprechenden Stickstoffatom ein Wasserstoffatom oder einen anderen Substituenten aufweisen. Beispiele umschließen Pyrrol, Imidazol, Pyrrolidin, Morpholin, Piperazinreste usw.. Diese stickstoffhaltigen heterocyclischen Reste können auch über das Ring-Stickstoffatom gebunden sein, insbesondere wenn der entsprechende heterocyclische Rest an ein Kohlenstoffatom gebunden ist. Beispielsweise kann ein Thienylrest als 2-Thienyl oder 3-Thienyl vorliegen, ein Piperidinylrest als 1-Piperidinyl (Piperidino), 2-Piperidinyl, 3-Piperidinyl oder 4-Piperidinyl. Geeignete stickstoffhaltige Heterocyclen können auch als N-Oxide oder als quarternäre Salze, die ein Gegenion aufweisen, das von einer physiologisch annehmbaren Säure abgeleitet ist, vorliegen. Beispielsweise können Pyridylreste als Pyridin-N-oxide vorliegen. Geeignete schwefelhaltige Heterocyclen können auch als S-Oxid oder S-S-Dioxid vorliegen.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind. Die Bezeichnung Aryl umfasst in der vorliegenden Erfindung auch beispielsweise bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl.

Arylalkyl (wie Aryl-(C₁-C₆-alkyl)-) bedeutet, dass ein Alkylrest (wie C₁-C₆-Alkyl) wiederum mit einem Arylrest substituiert ist. Heteroarylalkyl (wie Heteroaryl-(C₁-C₆-alkyl)-) bedeutet, dass ein Alkylrest (wie C₁-C₆-Alkyl) wiederum mit einem Heteroarylrest substituiert ist. Heterocyclylalkyl (wie Heterocyclyl-(C₁-C₆-alkyl)-) bedeutet, dass ein Alkylrest (wie C₁-C₆-Alkyl) wiederum mit einem Heterocyclylrest substituiert ist. Für die Definitionen und Substitutionsmöglichkeiten von Alkyl, Heteroaryl, Heterocyclyl und Aryl wird auf die vorstehenden Definitionen verwiesen.

Halogen ist Fluor, Chlor, Brom oder Iod, ist bevorzugt Fluor, Chlor, oder Brom, ist mehr bevorzugt Fluor oder Chlor.

Die vorliegende Erfindung schließt alle stereo-isomeren Formen von Verbindungen der Formel I mit ein. Asymmetrische Kohlenstoffatome in Verbindungen der Formel I können unabhängig voneinander S-Konfigurationen oder R-Konfigurationen aufweisen. Die Erfindung schließt alle möglichen Enantiomere und Diastereomere und Mischungen aus zwei oder mehr Stereo-Isomeren, zum Beispiel Mischungen aus Enantiomeren und/oder Diastereomeren, in allen Mengen und Verhältnissen mit ein. Somit können Verbindungen der vorliegenden Erfindung, die als Enantiomere existieren, in Enantiomeren-Reinform, sowohl als rechtsdrehende als auch linksdrehende Antipoden, in Form von Racematen und in Form von Mischungen der zwei Enantiomeren in allen Verhältnissen vorliegen. Im Fall von cis/trans-Isomeren schließt die Erfindung sowohl die Cis-Form, als auch die Trans-Form sowie Mischungen von diesen Formen in allen Verhältnissen mit ein. All diese Formen sind Gegenstand der vorliegenden Erfindung. Die Herstellung der einzelnen Stereo-Isomeren kann, falls gewünscht, durch Trennung eines Gemisches mit herkömmlichen Methoden, zum Beispiel durch Chromatographie oder Kristallisation, durch die Verwendung von stereochemisch einheitlichen Ausgangsmaterialien zur Synthese oder durch stereoselektive Synthese erfolgen. Alternativ kann auch einer Derivatisierung vor der Trennung der Stereo-Isomeren durchgeführt werden. Die Trennung eines Gemisches aus Stereo-Isomeren kann mit den Verbindungen der Formel I oder mit den entsprechenden Zwischenprodukten während der Synthese durchgeführt werden. Weiterhin umfasst die vorliegende Erfindung auch alle tautomeren Formen von Verbindungen gemäß Formel (I), insbesondere Keto/Enoltautomerie, d.h. die entsprechenden Verbindungen können entweder in ihrer Keto-Form oder in ihrer Enolform oder in Mischungen davon in allen Verhältnissen vorliegen.

Sofern die Verbindungen Formel I eine oder mehrere saure oder basische Gruppen enthalten, umfasst die vorliegende Erfindung auch die entsprechend physiologisch oder toxikologisch verträglichen Salze.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Theophyllinessigsäure, Methylen-bis-b-oxynaphthon-, Benzolsulfon-, Benzoe-, Zitronen-, Ethansulfon-, Salicyl-, Fumar-, Glucon-, Glykol-, Isethion-, Milch-, Lactobion-, Malein-, Äpfel-, Methansulfon-, Bernstein-, p-Toluolsulfon-, Wein- und Trifluoressigsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Sofern die Verbindungen der Formel I gleichzeitig saure und basische Gruppen in demselben Molekül enthalten, schließt die vorliegende Erfindung - zusätzlich zu den vorher aufgeführten Salzformen - auch innere Salze oder Betaine (Zwitterionen) mit ein.
Die entsprechenden Salze der Verbindungen gemäß Formel I können durch herkömmliche Methoden, die dem Fachmann bekannt sind, erhalten werden, beispielsweise durch Umsetzung mit einer organischen oder anorganischen Säure oder Base in einem Lösungsmittel oder Dispergiermittel, oder durch Anionen- oder Kationenaustausch mit anderen Salzen.

Die vorliegende Erfindung schließt darüber hinaus alle Solvate von Verbindungen der Formel (1) mit ein, beispielsweise Hydrate oder Addukte mit Alkohol, aktive Metaboliten von Verbindungen der Formel I sowie Derivate, die eine physiologisch annehmbare und abspaltbare Gruppe enthalten, beispielsweise Ester oder Amide.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht und sind ebenfalls Gegenstand der vorliegenden Erfindung.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Bevorzugte Verbindungen der allgemeinen Formel I sind diejenigen Verbindungen, in denen einer, mehrere oder alle der vorstehend aufgeführten Substituenten R1 bis R5, X, Ar, Heteroaryl, Heterocyclyl und Aryl unabhängig voneinander die nachstehend aufgeführten Bedeutungen (Definitionen) haben, wobei alle möglichen Kombinationen von bevorzugten, mehr bevorzugten und besonders bevorzugten Bedeutungen (Definitionen), ebenso in Kombination mit den Substituenten in ihrer obengenannten Bedeutung, Gegenstand der vorliegenden Erfindung sind.
- X: ist bevorzugt O, NH oder N(C₁-C₃-Alkyl);
- X: ist mehr bevorzugt NH oder N(C₁-C₃-Alkyl) und besonders bevorzugt NH oder N-Methyl;
- R1: ist bevorzugt Fluor, -OCF₃ oder C₁-C₄-Alkyl;
- R1: ist mehr bevorzugt C₁-C₃-Alkyl und besonders bevorzugt Ethyl;
- R2: ist bevorzugt Wasserstoff, Fluor, -OCF₃ oder C₁-C₄-Alkyl;
- R2: ist mehr bevorzugt Wasserstoff;
- R3: ist bevorzugt Fluor, -OCF₃ oder C₁-C₄-Alkyl;
- R3: ist mehr bevorzugt C₁-C₃-Alkyl und besonders bevorzugt Methyl;
- Ar: ist bevorzugt unsubstituiertes oder einfach substituiertes Phenyl oder Heteroaryl, wobei die Substituenten ausgewählt sind aus der Gruppe bestehend aus:
Fluor, Chlor, -CF₃, -OCF₃, -CN, -C(O)(C₁-C₃-Alkyl), NH₂, -NHC(O)(C₁-C₃-Alkyl), Hydroxy, Oxo, C₁-C₃-Alkyl, C₁-C₃-Alkoxy, -NH(C₁-C₃-Alkyl), -N(C₁-C₃-Alkyl)₂, -SO₂(C₁-C₃-Alkyl), Heterocyclyl, Heteroaryl, Aryl, -O-Aryl, -O-Heteroaryl, -CH₂-NR4R5, -SO₂NR4R5, und -C(O)NR4R5,
wobei der Substituent C₁-C₃-Alkyl mit C₁-C₃-Alkoxy, Fluor, Chlor, Trifluormethyl, Trifluormethoxy, Aryl, Heteroaryl, Heterocyclyl oder OH zumindest einfach substituiert sein kann,
und die Substituenten Aryl, Heteroaryl und Heterocyclyl mit C₁-C₃-Alkyl, C₁-C₃-Alkoxy, Fluor, Chlor, Trifluormethyl, Trifluormethoxy oder OH zumindest einfach substituiert sein können;
- Ar: ist mehr bevorzugt unsubstituiertes oder zumindest einfach substituiertes Phenyl, Indanyl, Naphthyl, Indolyl, Benzofuranyl, Benzimidazolyl, Furanyl, Thienyl, Imidazolyl, Pyrimidinyl, Pyrazolyl, Naphtyl, Isochinolinyl, Pyridinyl, Chinolinyl, 2,3-Dihydroindolyl, 5,6,7,8-Tetrahydronaphtyl
wobei die Substituenten ausgewählt sind aus der Gruppe bestehend aus: Fluor, Chlor, -CF₃, -OCF₃, -CN, -C(O)(C₁-C₃-Alkyl), NH₂, -NHC(O)(C₁-C₃-Alkyl), Hydroxy, Oxo, C₁-C₃-Alkyl, C₁-C₃-Alkoxy, -NH(C₁-C₃-Alkyl), -N(C₁-C₃-Alkyl)₂, -SO₂(C₁-C₃-Alkyl), Heteroaryl, Phenyl, -O-Phenyl, -O-Heteroaryl, -CH₂-NR4R5, -SO₂NR4R5, und -C(O)NR4R5,
wobei der Substituent C₁-C₃-Alkyl mit C₁-C₃-Alkoxy, Fluor, Chlor, Trifluormethyl, Trifluormethoxy, Phenyl, Heteroaryl oder OH zumindest einfach substituiert sein kann,
und die Substituenten Phenyl und Heteroaryl mit C₁-C₃-Alkyl, C₁-C₃-Alkoxy, Fluor, Chlor, Trifluormethyl, Trifluormethoxy oder OH zumindest einfach substituiert sein können;
- Ar: ist besonders bevorzugt
unsubstituiertes oder einfach substituiertes Phenyl, Pyridyl, Chinolinyl, Indanyl, 2,3-Dihydroindolyl, 5,6,7,8-Tetrahydronaphtyl,
wobei die Substituenten ausgewählt sind aus der Gruppe bestehend aus: -CN, -C(O)(C₁-C₃-Alkyl), -NHC(O)(C₁-C₃-Alkyl), Oxo, C₁-C₃-Alkyl, C₁-C₃-Alkoxy, -SO₂(C₁-C₃-Alkyl), Heteroaryl, -O-Heteroaryl, -CH₂-NR4R5, -SO₂NR4R5, und -C(O)NR4R5,
wobei der Substituent C₁-C₃-Alkyl mit Heteroaryl oder OH einfach substituiert sein kann,
und der Substituenten Heteroaryl mit C₁-C₃-Alkyl einfach substituiert sein kann;
- R4: ist bevorzugt Wasserstoff oder C₁-C₃-Alkyl;
- R4: ist mehr bevorzugt Wasserstoff
- R5: ist bevorzugt ausgewählt aus der Gruppe bestehend aus; Wasserstoff; unsubstituiertes oder zumindest einfach substituiertes C₁-C₆-Alkyl, Cyclohexenyl, Indanyl, Phenyl, Pyrrolidinyl, Pyrrolyl, Pyrazolyl, Furanyl und Piperidinyl,
wobei die Substituenten ausgewählt sind aus der Gruppe bestehend aus: Fluor, -CN, -C(O)NH₂, -O-Phenyl, -C(O)-Phenyl, -N(CH₃)₂, C₁-C₃-Alkyl, C₁-C₃-Alkoxy, Hydroxy, unsubstituiertes oder zumindest monosubstituiertes Phenyl, Pyridinyl, Thienyl, Pyrimidinyl, Imidazolyl, Furanyl, Indolyl, Benzimidazolyl, Pyrazolyl, Morpholinyl, Pyrrolidinyl, 1,3-Benzodioxolyl, Piperidinyl, Tetrahydropyranyl, Triazolyl, Thiazolyl, Thiazolidinyl, Isoxazolyl und Dihydroisoxazolyl, deren Substituenten sind wiederum ausgewählt aus der Gruppe bestehend aus: Fluor, Chlor, Oxo, CF₃, -OCF₃, -NO₂, Phenyl, Pyridinyl, -NHC(O)CH₃, -COOH, Hydroxy, C₁-C₃-Alkyl, C₁-C₃-Alkoxy, -SO₂NH₂, -C(O)NH₂ und -N(CH₃)₂;
- R5: ist mehr bevorzugt ausgewählt aus der Gruppe bestehend aus; Wasserstoff; unsubstituiertes oder einfach substituiertes C₁-C₆-Alkyl, Cyclohexenyl, Indanyl, Phenyl, Pyrrolidinyl, Pyrrolyl, Pyrazolyl, Furanyl und Piperidinyl,
wobei die Substituenten ausgewählt sind aus der Gruppe bestehend aus: Fluor, -CN, -C(O)NH₂, -O-Phenyl, -C(O)-Phenyl, -N(CH₃)₂, C₁-C₃-Alkyl, C₁-C₃-Alkoxy, Hydroxy, unsubstituiertes oder zumindest monosubstituiertes Phenyl, Pyridinyl, Thienyl, Pyrimidinyl, Imidazolyl, Furanyl, Indolyl, Benzimidazolyl, Pyrazolyl, Morpholinyl, Pyrrolidinyl, 1,3-Benzodioxolyl, Piperidinyl, Tetrahydropyranyl, Triazolyl, Thiazolyl, Thiazolidinyl, Isoxazolyl und Dihydroisoxazolyl, deren Substituenten sind wiederum ausgewählt aus der Gruppe bestehend aus: Fluor, Chlor, Oxo, CF₃, -OCF₃, -NO₂, Phenyl, Pyridinyl, -NHC(O)CH₃, -COOH, Hydroxy, C₁-C₃-Alkyl, C₁-C₃-Alkoxy, -SO₂NH₂, -C(O)NH₂ und -N(CH₃)₂;
- R5: ist besonders bevorzugt Wasserstoff, C1-C3 Alkyl oder Pyridinyl
- R4 und R5: bilden bevorzugt zusammen mit dem Stickstoffatom, an das sie gebunden sind, einen Rest ausgewählt aus der Gruppe bestehend aus: unsubstituiertes oder zumindest einfach substituiertes Piperidinyl, Pyrrolidinyl, Morpholinyl und Piperazinyl,
wobei die Substituenten ausgewählt sind aus der Gruppe bestehend aus: Fluor, -C(O)(C₁-C₃-Alkyl), Oxo, C₁-C₃-Alkyl, Hydroxy, unsubstituiertes oder zumindest einfach substituiertes Phenyl, Imidazolyl, Pyridinyl, Pyrimidinyl, Piperidinyl und Pyrrolidinyl, deren Substituenten sind wiederum Fluor oder C₁-C₃-Alkyl; .
- R4 und R5: bilden mehr bevorzugt zusammen mit dem Stickstoffatom, an das sie gebunden sind, einen Pyrrolidinylrest;
- Aryl: ist bevorzugt Phenyl, Indanyl oder Naphthyl;
- Aryl: ist mehr bevorzugt Phenyl oder Indanyl;
- Aryl: ist besonders bevorzugt Phenyl;

Heteroaryl ist bevorzugt Pyridinyl, Thienyl, Pyrimidinyl, Imidazolyl, Furanyl, Indolyl, Benzimidazolyl, Pyrazolyl, 1,3-Benzodioxolyl, Triazolyl, Thiazolyl, Isoxazolyl, Pyrrolyl, Pyrazinyl, Oxazolyl, Pyridazinyl, Chinolinyl, Isochinolinyl, Benzofuranyl, 3-Oxo-1,3-dihydroisobenzofuranyl, 2,3-Dihydroindolyl oder 4,5,6,7-Tetrahydrobenzothiazolyl; .

Heteroaryl ist mehr bevorzugt Pyridinyl, Chinolinyl, Indolyl, Benzofuranyl, Thienyl, Pyrimidinyl, Imidazolyl, Furanyl, Benzimidazolyl, Pyrazolyl, Thiazolyl, Isochinolinyl, Pyrrolyl oder 2,3-Dihydroindolyl;

Heteroaryl ist besonders bevorzugt Pyridinyl, Imidazolyl; oder Pyrimidinyl;

Heterocyclyl ist bevorzugt Morpholinyl, Pyrrolidinyl, Piperidinyl, Tetrahydropyranyl, Thiazolidinyl, Dihydroisoxazolyl, Piperazinyl oder Tetrahydrofuranyl;

Heterocyclyl ist mehr bevorzugt Morpholinyl, Pyrrolidinyl, Piperidinyl oder Piperazinyl; Heterocyclyl ist besonders bevorzugt Pyrrolidinyl.

Die Erfindung betrifft auch Verbindungen der Formel I, in denen bedeuten:
- X: O, NH oder N(C₁-C₃-Alkyl);
- R1: Fluor, -OCF₃ oder C₁-C₄-Alkyl;
- R2: Wasserstoff, Fluor, -OCF₃ oder C₁-C₄-Alkyl;
- R3: Fluor, -OCF₃ oder C₁-C₄-Alkyl;
- Ar: unsubstituiertes oder einfach substituiertes Phenyl oder Heteroaryl, wobei die Substituenten ausgewählt sind aus der Gruppe bestehend aus: Fluor, Chlor, -CF₃, -OCF₃, -CN, -C(O)(C₁-C₃-Alkyl), NH₂, -NHC(O)(C₁-C₃-Alkyl), Hydroxy, Oxo, C₁-C₃-Alkyl, C₁-C₃-Alkoxy, -NH(C₁-C₃-Alkyl), -N(C₁-C₃-Alkyl)₂, -SO₂(C₁-C₃-Alkyl), Heterocyclyl, Heteroaryl, Aryl, -O-Aryl, -O-Heteroaryl, -CH₂-NR4R5, -SO₂NR4R5, und -C(O)NR4R5,
wobei der Substituent C₁-C₃-Alkyl mit C₁-C₃-Alkoxy, Fluor, Chlor, Trifluormethyl, Trifluormethoxy, Aryl, Heteroaryl, Heterocyclyl oder OH zumindest einfach substituiert sein kann,
und die Substituenten Aryl, Heteroaryl und Heterocyclyl mit C₁-C₃-Alkyl, C₁-C₃-Alkoxy, Fluor, Chlor, Trifluormethyl, Trifluormethoxy oder OH zumindest einfach substituiert sein können;
- R4: Wasserstoff oder C₁-C₃-Alkyl;
- R5: ist ausgewählt aus der Gruppe bestehend aus; Wasserstoff; unsubstituiertes oder zumindest einfach substituiertes C₁-C₆-Alkyl, Cyclohexenyl, Indanyl, Phenyl, Pyrrolidinyl, Pyrrolyl, Pyrazolyl, Furanyl und Piperidinyl,
wobei die Substituenten ausgewählt sind aus der Gruppe bestehend aus: Fluor, -CN, -C(O)NH₂, -O-Phenyl, -C(O)-Phenyl, -N(CH₃)₂, C₁-C₃-Alkyl, C₁-C₃-Alkoxy, Hydroxy, unsubstituiertes oder zumindest monosubstituiertes Phenyl, Pyridinyl, Thienyl, Pyrimidinyl, Imidazolyl, Furanyl, Indolyl, Benzimidazolyl, Pyrazolyl, Morpholinyl, Pyrrolidinyl, 1,3-Benzodioxolyl, Piperidinyl, Tetrahydropyranyl, Triazolyl, Thiazolyl, Thiazolidinyl, Isoxazolyl und Dihydroisoxazolyl, deren Substituenten sind wiederum ausgewählt aus der Gruppe bestehend aus: Fluor, Chlor, Oxo, CF₃, -OCF₃, -NO₂, Phenyl, Pyridinyl, -NHC(O)CH₃, -COOH, Hydroxy, C₁-C₃-Alkyl, C₁-C₃-Alkoxy, -SO₂NH₂, -C(O)NH₂ und -N(CH₃)₂; oder
- R4 und R5: bilden zusammen mit dem Stickstoffatom, an das sie gebunden sind, einen Rest ausgewählt aus der Gruppe bestehend aus: unsubstituiertes oder zumindest einfach substituiertes Piperidinyl, Pyrrolidinyl, Morpholinyl und Piperazinyl,
wobei die Substituenten ausgewählt sind aus der Gruppe bestehend aus: Fluor, -C(O)(C₁-C₃-Alkyl), Oxo, C₁-C₃-Alkyl, Hydroxy, unsubstituiertes oder zumindest einfach substituiertes Phenyl, Imidazolyl, Pyridinyl, Pyrimidinyl, Piperidinyl und Pyrrolidinyl, deren Substituenten sind wiederum Fluor oder C₁-C₃-Alkyl;
- Aryl: Phenyl, Indanyl oder Naphthyl;
- Heteroaryl: Pyridinyl, Thienyl, Pyrimidinyl, Imidazolyl, Furanyl, Indolyl, Benzimidazolyl, Pyrazolyl, 1,3-Benzodioxolyl, Triazolyl, Thiazolyl, Isoxazolyl, Pyrrolyl, Pyrazinyl, Oxazolyl, Pyridazinyl, Chinolinyl, Isochinolinyl, Benzofuranyl, 3-Oxo-1,3-dihydroisobenzofuranyl, 2,3-Dihydroindolyl oder 4,5,6,7-Tetrahydrobenzothiazolyl;
- Heterocyclyl: Morpholinyl, Pyrrolidinyl, Piperidinyl, Tetrahydropyranyl, Thiazolidinyl, Dihydroisoxazolyl, Piperazinyl oder Tetrahydrofuranyl;
sowie deren physiologisch verträglichen Salze.

Die Erfindung betrifft ferner Verbindungen der Formel 1, worin bedeuten:
- X: NH oder N(C₁-C₃-Alkyl);
- R1: C₁-C₃-Alkyl;
- R2: Wasserstoff;
- R3: C₁-C₃-Alkyl;
- Ar: unsubstituiertes oder einfach substituiertes Phenyl, Indolyl, Benzofuranyl, Benzimidazolyl, Furanyl, Thienyl, Imidazolyl, Pyrimidinyl, Pyrazolyl; Isochinolinyl, Pyridinyl, Chinolinyl, oder 2,3-Dihydroindolyl,
wobei die Substituenten ausgewählt sind aus der Gruppe bestehend aus: Fluor, Chlor, -CF₃, -OCF₃, -CN, -C(O)(C₁-C₃-Alkyl), NH₂, -NHC(O)(C₁-C₃-Alkyl), Hydroxy, Oxo, C₁-C₃-Alkyl, C₁-C₃-Alkoxy, -NH(C₁-C₃-Alkyl), -N(C₁-C₃-Alkyl)₂, -SO₂(C₁-C₃-Alkyl), Heteroaryl, Phenyl, -O-Phenyl, -O-Heteroaryl, -CH₂-NR4R5, -SO₂NR4R5, und -C(O)NR4R5,
wobei der Substituent C₁-C₃-Alkyl mit C₁-C₃-Alkoxy, Fluor, Chlor, Trifluormethyl, Trifluormethoxy, Phenyl, Heteroaryl oder OH zumindest einfach substituiert sein kann,
und die Substituenten Phenyl und Heteroaryl mit C₁-C₃-Alkyl, C₁-C₃-Alkoxy, Fluor, Chlor, Trifluormethyl, Trifluormethoxy oder OH zumindest einfach substituiert sein können;
- R4: Wasserstoff
- R5: ist ausgewählt aus der Gruppe bestehend aus;
Wasserstoff; unsubstituiertes oder einfach substituiertes C₁-C₆-Alkyl, Cyclohexenyl, Indanyl, Phenyl, Pyrrolidinyl, Pyrrolyl, Pyrazolyl, Furanyl und Piperidinyl,
wobei die Substituenten ausgewählt sind aus der Gruppe bestehend aus: Fluor, -CN, -C(O)NH₂, -O-Phenyl, -C(O)-Phenyl, -N(CH₃)₂, C₁-C₃-Alkyl, C₁-C₃-Alkoxy, Hydroxy, unsubstituiertes oder zumindest monosubstituiertes Phenyl, Pyridinyl, Thienyl, Pyrimidinyl, Imidazolyl, Furanyl, Indolyl, Benzimidazolyl, Pyrazolyl, Morpholinyl, Pyrrolidinyl, 1,3-Benzodioxolyl, Piperidinyl, Tetrahydropyranyl, Triazolyl, Thiazolyl, Thiazolidinyl, Isoxazolyl und Dihydroisoxazolyl, deren Substituenten sind wiederum ausgewählt aus der Gruppe bestehend aus: Fluor, Chlor, Oxo, CF₃, -OCF₃, -NO₂, Phenyl, Pyridinyl, -NHC(O)CH₃, -COOH, Hydroxy, C₁-C₃-Alkyl, C₁-C₃-Alkoxy, -SO₂NH₂, -C(O)NH₂ und -N(CH₃)₂; oder
- R4 und R5: bilden zusammen mit dem Stickstoffatom, an das sie gebunden sind, einen unsubstituierten oder zumindest einfach substituierten Pyrrolidinylrest,
wobei die Substituenten ausgewählt sind aus der Gruppe bestehend aus: C₁-C₃-Alkyl und Hydroxy;
- Aryl: Phenyl oder Indanyl;
- Heteroaryl: Pyridinyl, Chinolinyl, Indolyl, Benzofuranyl, Thienyl, Pyrimidinyl, Imidazolyl, Furanyl, Benzimidazolyl, Pyrazolyl, Thiazolyl, lsochinolinyl, Pyrrolyl oder 2,3-Dihydroindolyl;
sowie deren physiologisch verträglichen Salze.

Die Erfindung betrifft auch Verbindungen der Formel I, in der bedeuten:
- X: NH oder N-Methyl;
- R1: Ethyl;
- R2: Wasserstoff;
- R3: Methyl;
- Ar: unsubstituiertes oder einfach substituiertes Phenyl, Indanyl, 5,6,7,8-Tetrahydronaphtyl, Pyridyl, Chinolinyl, oder 2,3-Dihydroindolyl,
wobei die Substituenten ausgewählt sind aus der Gruppe bestehend aus: -CN, -C(O)(C₁-C₃-Alkyl), -NHC(O)(C₁-C₃-Alkyl), Oxo, C₁-C₃-Alkyl, C₁-C₃-Alkoxy, -SO₂(C₁-C₃-Alkyl), Heteroaryl, -O-Heteroaryl, -CH₂-NR4R5, -SO₂NR4R5, und -C(O)NR4R5,
wobei der Substituent C₁-C₃-Alkyl mit Heteroaryl oder OH einfach substituiert sein kann,
und der Substituenten Heteroaryl mit C₁-C₃-Alkyl einfach substituiert sein kann;
- R4: Wasserstoff
- R5: Wasserstoff, C₁-C₃-Alkyl) oder Pyridinyl;
- R4 und R5: bilden zusammen mit dem Stickstoffatom, an das sie gebunden sind, einen Pyrrolidinylrest;
- Heteroaryl: Pyridinyl, Imidazolyl, oder Pyrimidinyl;
sowie deren physioligisch verträglichen Salze.

Ein Gegenstand der Erfindung sind auch Verbindungen der allgemeinen Formel I ausgewählt aus der Gruppe bestehend aus:
3-(5-Ethyl-2-methyl-6-oxo-1,6-dihydro-pyridin-3-ylamino)-benzönitril;
3-(5-Ethyl-2-methyl-6-oxo-1,6-dihydro-pyridin-3-ylamino)-benzamid;
5-(3-Acetyl-phenylamino)-3-ethyl-6-methyl-1H-pyridin-2-on;
N-[3-(5-Ethyl-2-methyl-6-oxo-1,6-dihydro-pyridin-3-ylamino)-phenyl]-acetamid;
3-Ethyl-6-methyl-5-(chinolin-3-ylamino)-1 H-pyridin-2-on;
4-(5-Ethyl-2-methyl-6-oxo-1,6-dihydro-pyridin-3-ylamino)-benzolsulfonamid;
3-Ethyl-6-methyl-5-[4-(pyridin-3-yloxy)-phenylamino]-1 H-pyridin-2-on;
3-Ethyl-5-(4-imidazol-1-yl-phenylamino)-6-methyl-1 H-pyridin-2-on;
5-(1-Acetyl-2,3-dihydro-1H-indol-6-ylamino)-3-ethyl-6-methyl-1H-pyridin-2-on;
4-(5-Ethyl-2-methyl-6-oxo-1,6-dihydro-pyridin-3-ylamino)-N-pyridin-2-yl-benzolsulfonamid;
3-Ethyl-6-methyl-5-(5-oxo-5,6,7,8-tetrahydro-naphthalin-2-ylamino)-1H-pyridin-2-on;
N-[6-(5-Ethyl-2-methyl-6-oxo-1,6-dihydro-pyridin-3-ylamino)-pyridin-3-yl]-acetamid;
6-(5-Ethyl-2-methyl-6-oxo-1,6-dihydro-pyridin-3-ylamino)-nicotinonitril;
3-Ethy1-6-methyl-5-(1-oxo-indan-5-ylamino)-1H-pyridin-2-on;
3-Ethyl-6-methyl-5-(pyridin-4-ylamino)-1H-pyridin-2-on;
3-Ethyl-6-methyl-5-(pyridin-2-ylamino)-1H-pyridin-2-on;
5-(2-Acetyl-phenylamino)-3-ethyl-6-methyl-1H-pyridin-2-on;
5-(4-Acetyl-phenylamino)-3-ethyl-6-methyl-1H-pyridin-2-on;
3-Ethyl-6-mothyl-5-(pyridin-3-ylamino)-1H-pyridin-2-on;
3-Ethyl-5-(3-methoxy-phenoxy)-6-methyl-1H-pyridin-2-on;
3-(5-Ethyl-2-methyl-6-oxo-1,6-dihydro-pyridin-3-yloxy)-benzonitril;
3-Ethyl-6-methyl-5-(3-methylaminomethyl-phenylamino)-1H-pyridin-2-on;
3-Ethyl-5-(3-hydroxymethyl-phenylamino)-6-methyl-1H-pyridin-2-on;
3-Ethyl-5-(3-methanesulfonyl-phenylamino)-6-methyl-1H-pyridin-2-on;
3-Ethyl-6-methyl-5-[3-(2-methyl-pyrimidin-4-yl)-phenylamino]-1H-pyridin-2-on;
3-Ethyl-6-methyl-5-(3-pyrrolidin-1-ylmethyl-phenylamino)-1H-pyridin-2-on;
3-Ethyl-5-[3-(1-hydroxy-ethyl)-phenylamino]-6-methyl-1H-pyridin-2-on;
3-Ethyl-6-methyl-5-(methyl-phenyl-amino)-1H-pyridin-2-on;
sowie deren physiologisch verträglichen Salze.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig- saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel 1 ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfasst die vorliegende Erfindung auch alle tautomeren Formen der Verbindungen der Formel I.

Die Verbindungen der Formel I sind durch unterschiedliche chemische Verfahren herstellbar, die ebenfalls zu der vorliegenden Erfindung gehören. Einige typische Wege sind in den unten als Schemata 1 bis 3 bezeichneten Reaktionssequenzen skizziert. Substituenten R sind jeweils wie oben angegeben definiert, sofern nachfolgend nichts anderes angegeben ist. Die Ausgangsverbindungen sowie die Zwischenverbindungen (Intermediate) sind entweder kommerziell erhältlich oder können mit dem Fachmann bekannten Verfahren hergestellt werden.

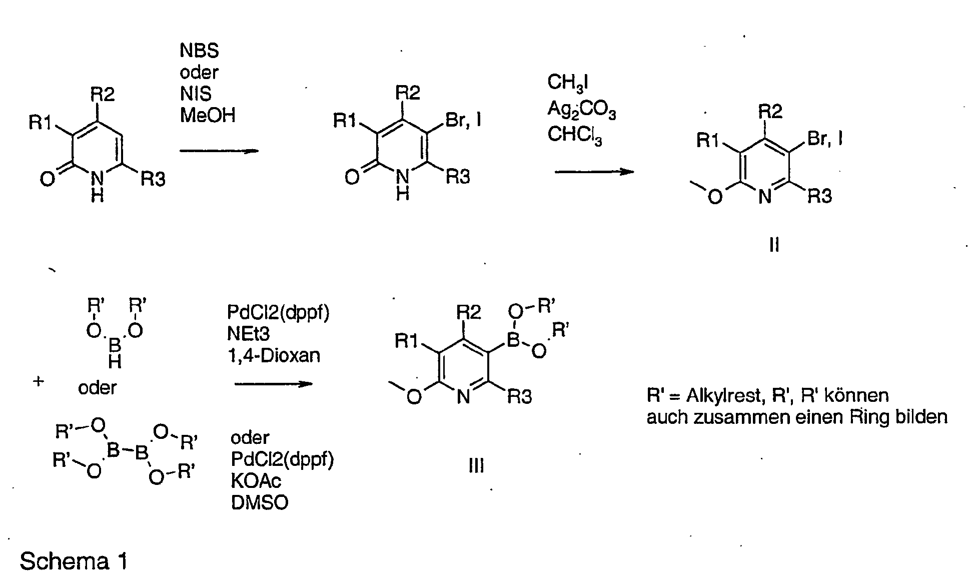

So erhält man beispielweise eine Verbindung der Formel I aus den Intermediaten II oder III (mögliche Darstellungen sind in Schema 1 beschrieben) gemäss Schemata 2 und 3.

Intermediat II kann aus einem entsprechend mit R1, R2 und R3 substituierten 2(1 H)-Pyridon durch Bromierung oder Iodierung (die z. Bsp. mit N-Bromsuccinimid oder N-lodsuccinimid in Methanol als Lösungsmittel durchgeführt werden kann) und anschliessende O-Alkylierung (z. Bsp. durch Methyliodid in Dichlormethan mit Zusatz von Silbercarbonat) dargestellt werden. Beispielsweise wird zur Herstellung von Verbindungen mit R1 gleich Ethyl, R2 gleich Wasserstoff und R3 gleich Methyl 3-Ethyl-6-methyl-2(1 H)-pyridon als Ausgangsmaterial gewählt. Entsprechend den gewünschten Resten für R1-R3 können andere entsprechend substituierte 2(1)H-Pyridone als Ausgangsmaterialen eingesetzt werden.

Aus Intermediat II kann durch Palladium-katalysierte Borylierung (z. Bsp. durch Umsetzung mit Bis(pinacolato)-diboron oder 4,4,5,5-Tetramethyl -1,3,2-dioxaborotan mit Palladiumdichlorid-1,1'-bis(diphenylphosphino)ferrocen als Katalysator und Kaliumacetat bzw. Triethylamin als Base in Dimethylsulfoxid bzw. 1,4-Dioxan als Lösungsmittel) Intermediat III hergestellt werden.

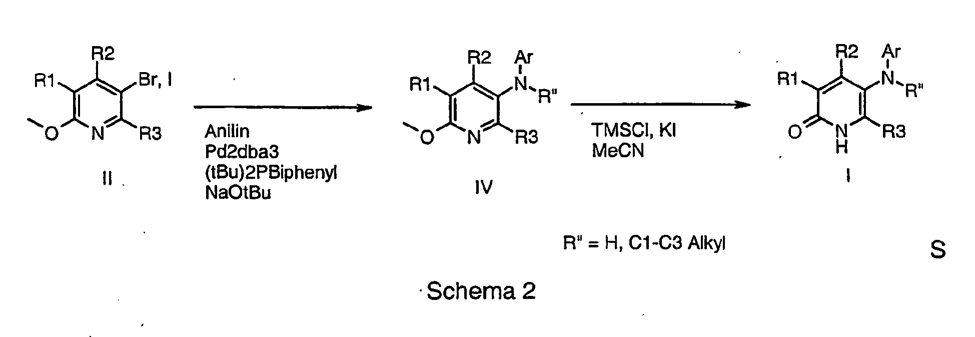

Verbindungen der Formel IV können durch Palladium-katalysierte Buchwald-Hartwig Aminierung mit einem Anilin dargestellt werden (Schema 2). Abspaltung der Methylgruppe aus den Verbindungen der Formel IV (z. Bsp. durch Trimethylchlorsilan und Kaliumiodid in Acetonitril) ergibt die Pyridone der Formel I.

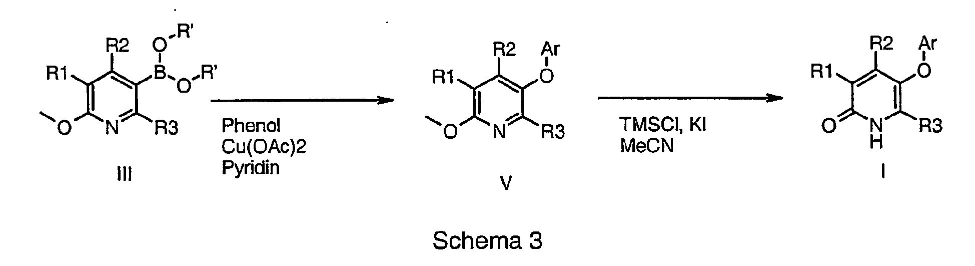

Zur Herstellung von Verbindungen der Formel V kann von Intermediat III ausgegangen werden das durch Kupfer-katalysierte Kupplung mit Phenolen (z. Bsp. in Pyridin) umgesetzt wird (Schema 3). Abspaltung der Methylgruppe aus den Verbindungen der Formel V (z. Bsp. durch Trimethylchlorsilan und Kaliumiodid in Acetonitril) ergibt die Pyridone der Formel I.

Die in Schema II und III eingesetzten Aniline und Phenole sind käuflich bzw. nach literaturbekannten Vorschriften synthetisierbar.

Bei allen Verfahrensweisen kann es angebracht sein, bei bestimmten Reaktionsvorschriften funktionelle Gruppen im Molekül zeitweilig zu schützen. Solche Schutzgruppen sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepasst werden.

Gegenstand der vorliegenden Erfindung ist auch die Verwendung von Verbindungen gemäß der Formel I als Arzneimittel beziehungsweise Medikament.

Die Verbindungen der allgemeinen Formel I sind PARP-Inhibitoren und eignen sich folglich zur Behandlung von Krankheiten, die zu PARP in Bezug stehen, dadurch verursacht werden, gefördert werden oder aus dessen Beteiligung resultieren.

Beispiele von Krankheiten, die mit den Verbindungen gemäß der vorliegenden Erfindung behandelt werden können, umfassen: Gewebeschaden resultierend aus Zellschädigung oder Zelltod aufgrund von Nekrose oder Apoptose, neuronal vermittelter Gewebeschaden oder Erkrankungen, zerebrale Ischämie, Kopftrauma, Schlaganfall, Reperfusionsschaden, neurologische Störungen und neurodegenerative Erkrankungen, vaskulärer Schlaganfall, Herz-Kreislauf-Störungen, Myokardinfarkt, Myokardischämie, experimentelle allergische Enzephalomyelitis (EAE), Multiple Sklerose (MS), Ischämie in Bezug zu Herzchirurgie, altersbezogene Makulardegeneration, Arthritis, Arterosklerose, Krebs, degenerative Erkrankungen des Skelettmuskels mit folgender replikativer Seneszenz, Diabetes und diabetische Herzmuskelerkrankungen.

Vorzugsweise werden die Verbindungen der vorliegenden Erfindung zur Behandlung von Krankheiten eingesetzt, die durch Ischämie oder Reperfusionsschäden verursacht werden. Krankheiten, die behandelt werden können, sind mehr bevorzugt ausgewählt , aus der Gruppe bestehend aus: zerebraler Ischämie, Reperfusionsschaden, Herz-Kreislauf-Störungen, Myokardinfarkt, Myokardischämie und Ischämie in Bezug zu Herzchirurgie.
Insbesondere können die Verbindungen der vorliegenden Erfindung zur Behandlung eines Myokardinfarktes verwendet werden.

In den vorstehenden Ausführungen umfasst der Begriff Behandlung auch die Prophylaxe, Therapie oder Heilung der vorgenannten Krankheiten.

Die erfindungsgemäßen Verbindungen der Formel und ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie der genannten Krankheitsbilder und ihre Verwendung zur Herstellung von Medikamenten dafür und von Medikamenten mit PARP inhibierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wässrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Myokardinfarkt, beispielsweise auf einer Intensivstation, kann auch eine parenterale Verabreichung durch Injektion oder Infusion, z. B. durch eine intravenöse Dauerinfusion, vorteilhaft sein.

### Experimenteller Teil

### Liste der Abkürzungen

- nBuLi: nButyllithium
- DMF: N,N-Dimethylformamid
- DMSO: Dimethylsulfoxid
- eq.: Moläquivalent
- MeCN: Acetonitril
- MeOH: Methanol
- NaOtBu: Natrium-tert. butanolat
- NBS: N-Bromsuccinimid
- NEt3: Triethylamin
- NIS: N-Iodsuccinimid
- PdCl2(dppf): 1,1'-Bis(diphenylphosphino)ferrocen-palladium(II)chlorid
- Pd2dba3: Tris(dibenzylidenaceton)dipalladium(0)
- RT: Raumtemperatur
- RP-HPLC: Umkehrphasen-Hochleistungschromatographie
- (tBu)2Pbiphenyl: Di(tert.-butyl) biphenylphosphin
- TFA: Trifluoressigsäure
- THF: Tetrahydrofuran
- TMSCI: Trimethylsilylchlorid
- Kl: Kaliumjodid

### Synthese der Halogenide der Formel II

Die Synthese gemäss Schema 1 wird anhand des Bromids demonstriert (R1 gleich Ethyl, R2 gleich Wasserstoff und R3 gleich Methyl):

### 3-Bromo-5-ethyl-6-methoxy-2-methylpyridin (Verbindung 1)

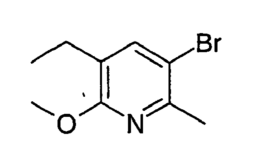

Eine Mischung von 3-Ethyl-6-methyl-1H-pyridin-2-on (23,92 g, 174,4 mmol) und N-Bromsuccinimid (32,67 g, 183,5 mmol) in Methanol (450 mL) wurde bei Raumtemperatur unter Stickstoff gerührt. Nach einigen Stunden bildete sich eine dicke Suspension die weitere 24h bei RT gerührt wurde. Die Reaktionsmischung wurde auf die Hälfte bis ein Drittel der ursprünglichen Volumens eingeengt und mit 200 mL Wasser verdünnt. Die Mischung wurde unter Rühren im Eisbad gekühlt und dann abgesaugt. Der Rückstand wurde mit kaltem Wasser gewaschen und bei 65°C getrocknet. Man erhielt 35,93 g (95%) von 5-Bromo-3-ethyl-6-methyl-1H-pyridin-2-on in Form eines beigen Pulvers.

5-Bromo-3-ethyl-6-methyl-1 H-pyridin-2-on (25 g, 115,7 mmol) wurde in 200 mL Chloroform unter Bildung einer orangen Lösung gelöst. Silbercarbonat (31,9 g, 115,7 mmol) wurde zugegeben und die Mischung kräftig gerührt. Diese Suspension wurde mit Methyliodid (10,8 mL, 1,5 mmol) versetzt und bei RT im Dunkeln unter Stickstoff gerührt. Nach 3 Tagen wurde der Ansatz filtriert und die Lösung am Rotationsverdampfer eingeengt. Der Rückstand wurde an Kieselgel mit n-Heptan als Laufmittel chromatographiert und ergab ein farbloses Öl (21,7 g, 82%).
MS: m/z = 230 und 232 (M+1)
1H-NMR (CDCl3): δ = 7,44 (s, 1H); 3,91 (s, 3H); 2,52 (q, 2H, J = 7,6 Hz); 2,50 (s, 3H); 1,16 (t, 3H, J = 7,6 Hz).

### Synthese der Borsäureester der Formel III

Die Synthese gemäss Schema 1 wird anhand des Borsäurepinakolesters demonstriert (R1 gleich Ethyl, R2 gleich Wasserstoff und R3 gleich Methyl):

### 3-Ethyl-2-methoxy-6-methyl-5-(4,4,5,5-tetramethyl-[1,3,2]dioxaborolan-2-yl)-pyridin (Verbindung 2)

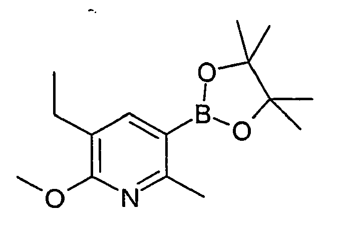

3-Bromo-5-ethyl-6-methoxy-2-methylpyridin (11,51 g, 50 mmol), 4,4,5,5-Tetramethyl-1,3,2-dioxaborotan (8,31 g, 64,95 mmol) und Triethylamin (21,84 mL, 164,4 mmol) wurden unter Argon in 100 mL Dioxan gelöst und anschliessend mit 1,1'Bis(diphenylphosphino)ferrocen-palladium(II)chlorid (1,94 g, 2,65 mmol) versetzt. Die Mischung wurde 18h bei 90°C gerührt, abgekühlt und mit Essigsäureethylester verdünnt und über Kieselgel filtriert. Die Lösung wurde auf 0°C gekühlt, mit Wasser versetzt und zweimal mit Essigsäureethylester extrahiert. Die getrocknete und eingeengte organische Phase wurde an Kieselgel chromatographiert bei 45°C im Vakuumtrockenschrank getrocknet. Man erhielt einen hellbeigen Feststoff (9,94 g, 72%).
MS: m/z = 278 (M+1)
1H-NMR (CDCl3): δ = 7,68 (s, 1H); 3,95 (s, 3H); 2,62 (s, 3H); 2,53 (q, 2H, J = 7,6 Hz); 1,33 (s, 12H); 1,16 (t, 3H, J = 7,6 Hz).

### Synthese der Anilinopyridine der Formel IV (Schema 2)

### Allgemeine Arbeitsvorschrift

135 mg (1,4 mmol) Natrium-tert-butylat wurden unter Argon in 2 mL Toluol gelöst, 230 mg (1 mmol) der Verbindung 1 und 2 mmol des jeweiligen Anilins wurdem zugesetzt, 10 min bei RT gerührt und anschliessend 48 mg (0,16 mmol) 2-(Di-tert-butylphosphino)biphenyl und 9,2 mg (0,01 mmol) Tris(dibenzylidenaceton)palladium(0) zugesetzt. Die Mischung wurde in der Mikrowelle (CEM Discover) bei 100°C 30 min zur Reaktion gebracht, dann mit Wasser und Essigester verdünnt und über eine Kieselgurkartusche (Varian Chem Elut) filtriert. Das gewünschte Produkt wurde in Ausbeuten zwischen 5 und 50% mittels RP-HPLC isoliert.

### Synthesevorschrift für Verbindung 3

### 3-(5-Ethyl-6-methoxy-2-methyl-pyridin-3-ylamino)-benzonitril (Verbindung 3)

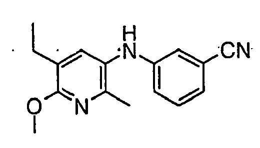

125 mg (1,3 mmol) Natrium-tert-butylat wurden unter Argon in 2 mL Toluol gelöst, 230 mg (1 mmol) der Verbindung 1 und 236 mg (2 mmol) 2-Aminobenzonitril wurden zugesetzt, 10 min bei RT gerührt und anschliessend 48 mg (0,16 mmol) 2-(Di-tert-butylphosphirio)biphenyl und 9,2 mg (0,01 mmol) Tris(dibenzylidenaceton)dipalladium(0) zugesetzt. Die Mischung wurde in der Mikrowelle (CEM Discover) bei 180°C 15 min zur Reaktion gebracht, dann mit Wasser und Essigester verdünnt, zweimal mit Essigester extrahiert, die organische Phase eingeengt und dann per RP-HPLC gereinigt. Man erhielt die 25 mg (9% d. Th.) der Titelverbindung.
MS: m/z = 268 (M+1)

Die folgenden Verbindungen der Formel IV wurden entsprechend der obigen allgemeinen Arbeitsvorschrift hergestellt. Dabei wurden für die Verbindungen 4 bis 21 und 24 bis 30 die folgenden Aniline eingesetzt:

4: 3-Aminobenzamid; 5: 3-Acetylanilin; 6: N-(3-Aminophenyl)-acetamid; 7: 3-Aminochinolin; 8: 4-Aminobenzolsulfonamid; 9: 4-(Pyridin-3-yloxy)-anilin; 10: 4-(Imidazol-1-yl)-anilin; 11: 1-(8-Amino-2,3-dihydro-indol-1-yl)-ethanon; 12: 4-Amino-N-pyridin-2-yl-benzolsulfonamid; 13: 6-Amino-3,4-dihydro-2H-naphthalin-1-on; 14: N-(6-Amino-pyridin-3-yl)-acetamid; 15: 6-Amino-nicotinnitril; 16: 5-Aminoindan-1-on; 17: 4-Aminopyridin; 18: 2-Aminopyridin; 19: 2-Acetylanilin; 20: 4-Acetylanilin; 21: 3-Aminopyridin; 24: 3-(Methylaminomethyl)-anilin; 25: 3-Hydroxymethylanilin; 26: 3-Methansulfonylanilin; 27: 3-(2-Methylpyrimidin-4-yl)-anilin; 28: 3-Pyrrolidin-1-ylmethylanilin; 29: 1-(3-Aminophenyl)-ethanol; 30: N-Methylanilin.

| Verbindung | Struktur | Masse m/z = |
|---|---|---|
| 4 | 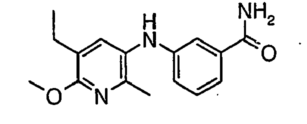 | 286 (M+1) |
| 5 | 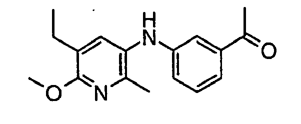 | 285 (M+1) |
| 6 | 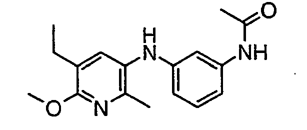 | 300 (M+1) |
| 7 | 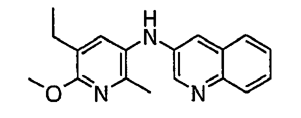 | 294 (M+1) |
| 8 | 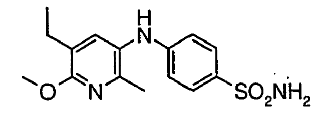 | 322 (M+1) |
| 9 | 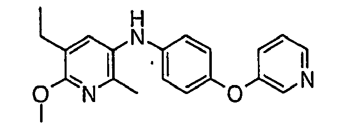 | 337 (M+1) |
| 10 | 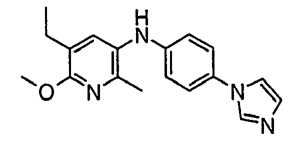 | 309 (M+1) |
| 11 | 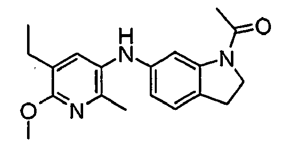 | 326(M+1) |
| 12 | 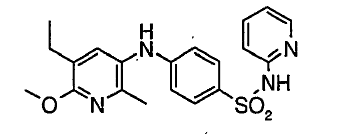 | 399(M+1) |
| 13 | 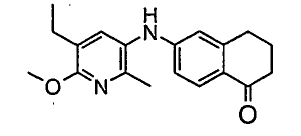 | 311 (M+1) |
| 14 | 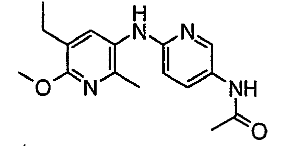 | 301 (M+1) |
| 15 | 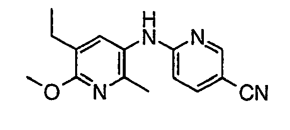 | 269 (M+1) |
| 16 | 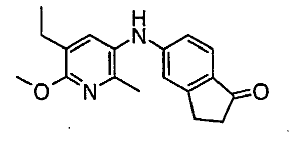 | 297 (M+1) |
| 17 | 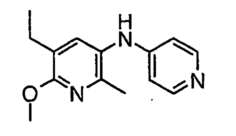 | 244 (M+1) |
| 18 | 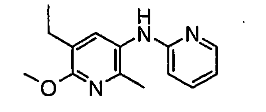 | 244 (M+1) |
| 19 | 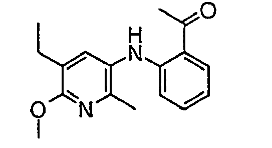 | 285 (M+1) |
| 20 | 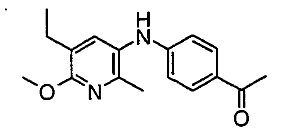 | 285 (M+1) |
| 21 | 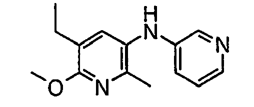 | 244 (M+1) |
| 24 | 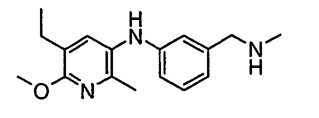 | 286 (M+1) |
| 25 | 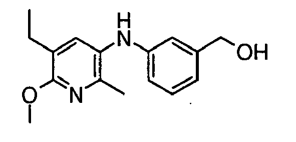 | 273 (M+1) |
| 26 | 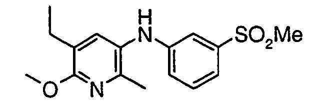 | 321 (M+1) |
| 27 | 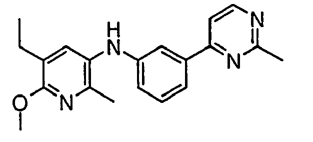 | 335 (M+1) |
| 28. | 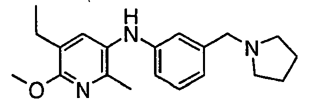 | 326 (M+1) |
| 29 | 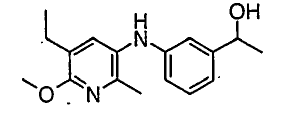 | 287 (M+1) |
| 30 | 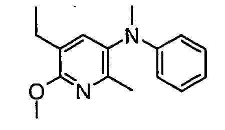 | 257 (M+1) |

### Synthese der Aryletherpyridine der Formel V (Schema 3)

### 3-Ethyl-2-methoxy-5-(3-methoxy-phenoxy)-6-methyl-pyridin (Verbindung 22)

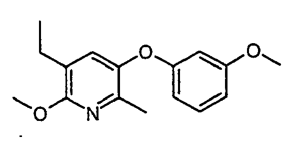

277 mg (1 mmol) der Verbindung 2, 62 mg (0.5 mmol) 3-Methoxyphenol und 90 mg (0,5 mmol) Kupfer(11)acetat wurden in 3 mL Dichlormethan gemischt, anschliessend wurde 253 mg (2.5 mmol) Triethylamin zugegeben und die Mischung bei RT 16 h gerührt. Die Mischung wurde mit Wasser und Dichlormethan versetzt, über eine Kieselgurkartusche filtriert und per RP-HPLC gereingt. Man erhielt 36 mg (26% d. Th) eines farblosen Öls.
MS: m/z = 274 (M+1)

### 3-(5-Ethyl-6-methoxy-2-methyl-pyridin-3-yloxy)-benzonitril (Verbindung 23)

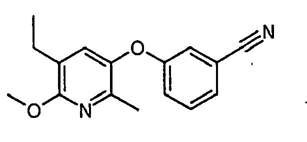

Analog wurden aus 60 mg (0.5 mmol) 3-Cyanophenol 35 mg (26% d. Th.) der oben genannten Titelverbindung isoliert.
MS: m/z = 269 (M+1)

### Synthese von Pyridonen der Formel I durch Entschützen der 2-Methoxypyridine der Formel IV und V

### Allgemeine Arbeitsvorschrift

Zu einer Mischung des 2-Methoxypyridins der Formel V oder V in wasserfreiem Acetonitril (3-5 mL/mmol) wurden 2 eq. Kaliumiodid und 2 eq. Trimethylchlorsilan unter Argon gegeben und die trübe Mischung 1-3 h bei 60-80°C erhitzt. Die Mischung wurde anschliessend auf RT abgekühlt und mit Wasser verdünnt. Das ausgefallene Produkt wurde abgesaugt, mit Wasser gewaschen und im Vakuumtrockenschrank bei 40°C getrocknet. Das Filtrat wurde mit Essigsäureethylester extrahiert und lieferte gelegentlich nach dem Einengen weiteres Produkt das - falls erforderlich - per HPLC gereinigt wurde

Entsprechend dieser Vorschriften wurden folgende Beispiele synthetisiert:

| Beispiel | Struktur und Verbindungsname | Masse m/z = | NMR (CDCl₃) δ = |
|---|---|---|---|
| 1 | 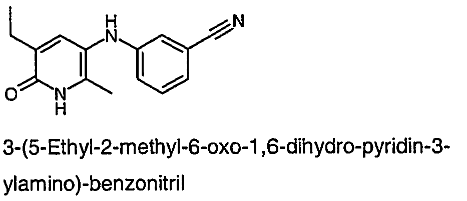 | 254 (M+1) | |
| 2 | 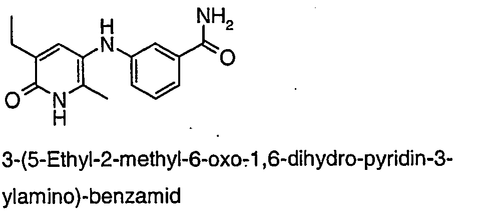 | 272 (M+1) | |
| 3 | 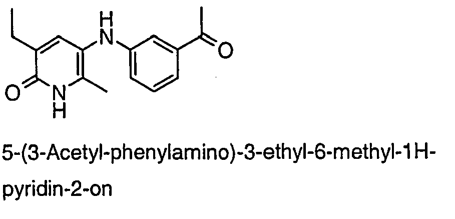 | 271 (M+1) | 15.3 (1 H, br s), 7.59 (1 H, s), 7.46 (1 H, m), 7.34 (1 H, m), 7.25(1 H, m), 6.84 (1 H, m), 2.63 (2H, q, J=7.4 Hz), 2.57 (3H, s), 2.43 (3H, s), 1.20 (3H, t, J=7.4 Hz). |
| 4 | 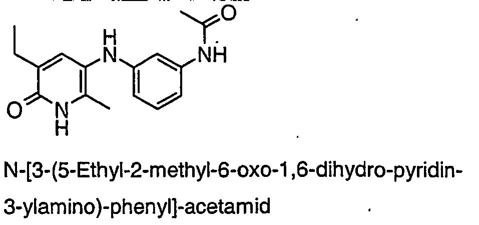 | 286 (M+1) | 14.8 (1H, br s), 7.59 (1 H, s), 7.25 (1 H, m), 7.14 (1H, m), 6.71 (1 H, m), 6.36 (1 H, m), 2.59 (2H, q, J=7.5 Hz), 2.40 (3H, s), 2.15 (3H, s), 1.19 (3H, t, J=7.5Hz). |
| 5 | 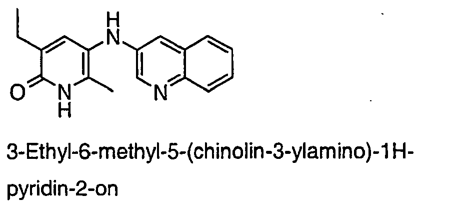 | 280 (M+1) | |
| 6 | 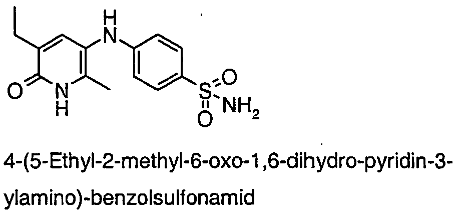 | 308 (M+1) | |
| 7 | 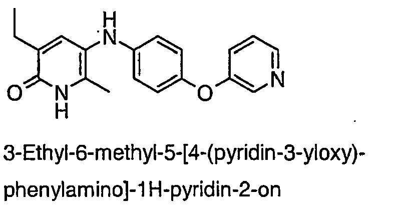 | 323 (M+1) | |
| 8 | 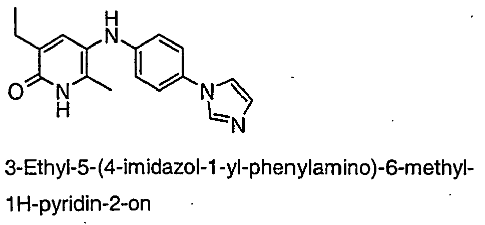 | 295 (M+1) | |
| 9 | 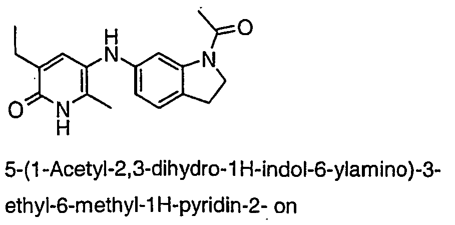 | 312(M+1) | |
| 10 | 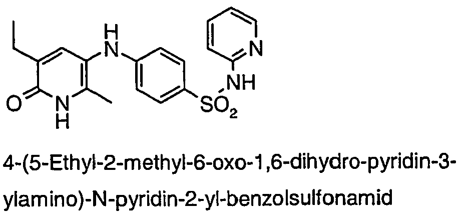 | 385 (M+1) | |
| 11 | 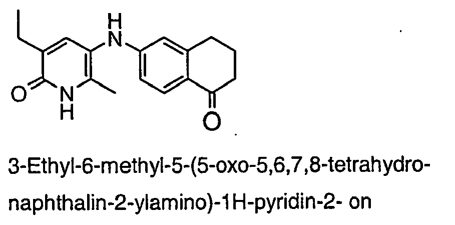 | 297 (M+1) | |
| 12 | 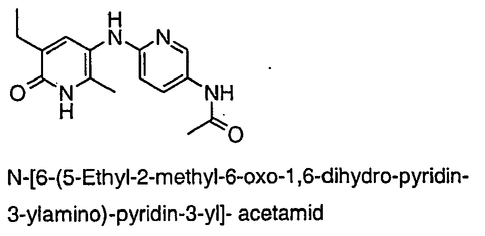 | 287 (M+1) | |
| 13 | 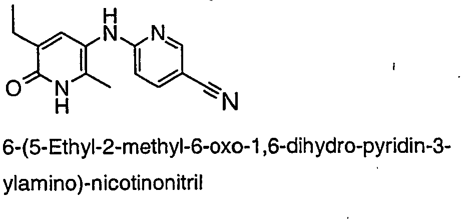 | 255 (M+1) | |
| 14 | 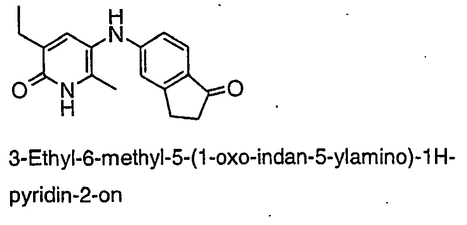 | 283 (M+1) | |
| 15 | 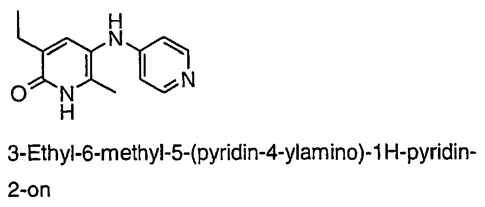 | 230 (M+1). | |
| 16 | 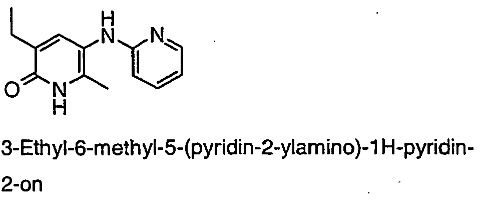 | 230 (M+1) | |
| 17 | 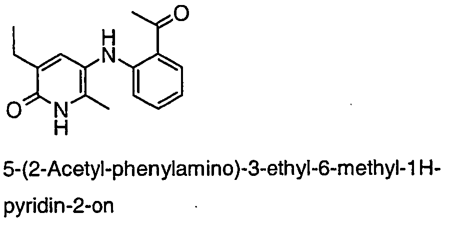 | 271 (M+1) | |
| 18 | 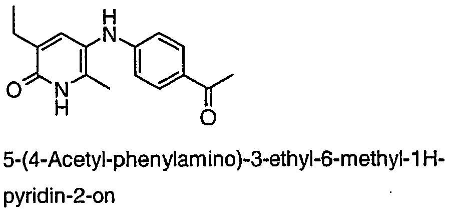 | 271 (M+1) | |
| 19 | 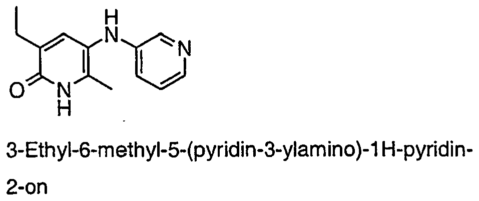 | 230 (M+1) | |
| 20 | 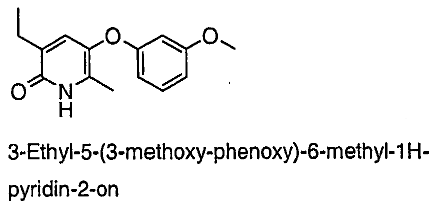 | 260 (M+1) | 11.7 (1H, s), 7.21 (1H, m), 7.08 (1 H, s), 6.61 (1 H, m), 6.44 (1 H, m) 6.39 (1 H, m), 3.72 (3H, s), 2.35 (2H, q, J=7.5 Hz), 2.01 (3H, s), 1.05 (3H, t, J=7.5 Hz). |
| 21 | 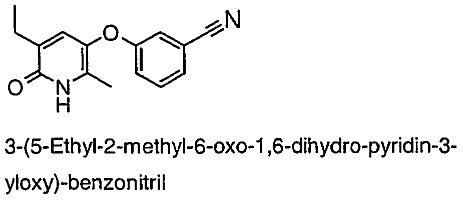 | 255 (M+1) | |
| 22 | 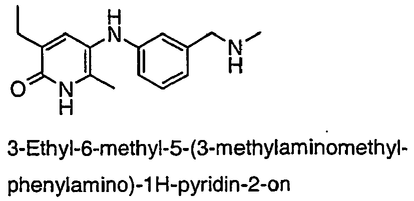 | 272 (M+1) | |
| 23 | 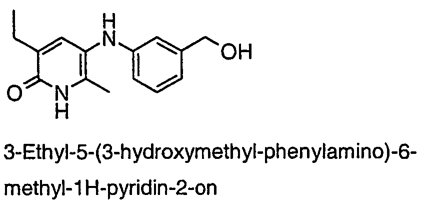 | 259 (M+1) | |
| 24 | 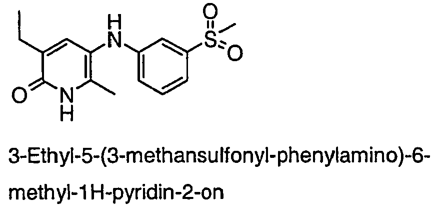 | 307 (M+1) | |
| 25 | 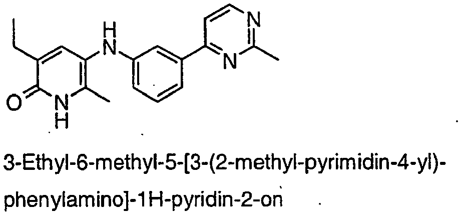 | 321 (M+1) | |
| 26 | 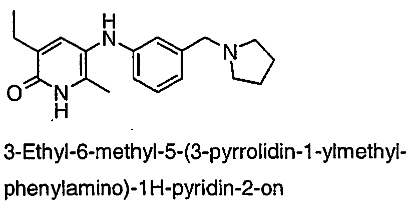 | 312 (M+1) | |
| 27 | 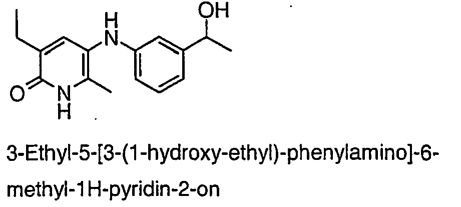 | 273 (M+1) | |
| 28 | 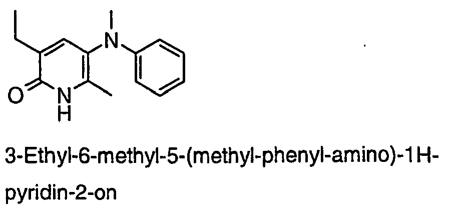 | 243 (M+1) | |

### Pharmakologische Untersuchungen

### PARP Enzymassay

Zur Bestimmung der halbmaximalen Inhibitorkonzentration werden die zu testenden Substanzen mit dem durch DNA aktivierten, rekombinant exprimierten und gereinigten PARP-1 Enzym inkubiert. Im Einzelnen werden verschiedene Konzentrationen der Testsubstanz in 50 µl Reaktionslösung, die 50 mM Tris, 5 mM MgCl₂, 1 mM DTT, 200 µM NAD, 0,1 mCi/ml Tritium-markiertes NAD, 0,1 mg/ml DNA, 0,1 mg/ml Histone, 2 µg/ml rekombinant exprimiertes humanes PARP-1 Enzym, pH=8,0 enthält, für 1 Stunde bei Raumtemperatur inkubiert. Die Reaktion wird durch Zugabe von 150 µl 20%ige Trichloressigsäure gestoppt und die radioaktiv markierten Proteinbestandteile gefällt. Nach einer 10-minütigen Inkubation auf Eis werden die markierten, unlöslichen Bestandteile durch einen Glasfaserfilter abgetrennt und nach dreimaligem Waschen mit 20%iger Trichloressigsäure die durch das PARP-1 Enzym eingebaute Radioaktivität durch Radiolumineszenz gemessen. Betrachtet man die so bestimmten Einbauraten in Abhängigkeit von der Konzentration der Testsubstanz, erhält man die halbmaximale Inhibitorkonzentration (IC₅₀) als die Konzentration der Testsubstanz, die die Einbaurate auf die Hälfte des maximal erreichbaren Wertes (Inkubation ohne Inhibitor) reduziert.

Auf diese Weise wurden für die nachfolgend aufgeführten Verbindungen folgende IC-50 Werte bestimmt:

| Bsp. | IC-50 [µM] | Bsp. | IC-50 [µM] |
|---|---|---|---|
| 1 | 9,0 | 13 | 3,9 |
| 2 | 2,2 | 15 | 9,5 |
| 3 | 1,3 | 18 | 4,4 |
| 4 | 0,8 | 19 | 9,2 |
| 6. | 9,8 | 20 | 4,8 |
| 7 | 4,7 | 23 | 9,8 |
| 9 | 3,4 | 24 | 6,1 |
| 10 | 6,2 | | |
| 12 | 1,1 | | |

## Patentansprüche

1. Verbindungen der Formel I 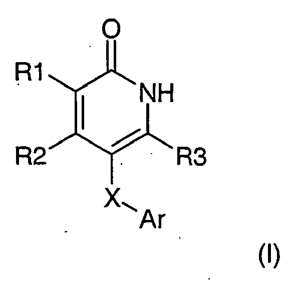 worin bedeuten:
R1 und R3 unabhängig voneinander Fluor, Methoxy, -OCF₃, C₂-C₃-Alkenyl oder C₁-C₄-Alkyl, das gegebenenfalls mit Chlor, Methoxy oder einem, zwei oder drei Fluoratomen substituiert ist;
R2 Wasserstoff, Fluor, Methoxy, -OCF₃, C₂-C₃-Alkenyl oder C₁-C₄-Alkyl, das gegebenenfalls mit Chlor, Methoxy oder einem, zwei oder drei Fluoratomen substituiert ist;
X O, S, NH oder N(C₁-C₃-Alkyl);
Ar unsubstituiertes oder zumindest einfach substituiertes Aryl oder Heteroaryl, wobei die Substituenten ausgewählt sind aus der Gruppe bestehend aus:
Fluor, Chlor, Brom, -CF₃, -OCF₃, -NO₂, -CN, -C(O)(C₁-C₆-Alkyl), NH₂, -NHC(O)(C₁-C₆-Alkyl), Hydroxy, Oxo, C₁-C₆-Alkyl, C₁-C₆-Alkoxy, -NH(C₁-C₆-Alkyl), -N(C₁-C₆-Alkyl)₂, -SO₂(C₁-C₆-Alkyl), Heterocyclyl, Heteroaryl, Aryl, -O-Aryl, -O-Heteroaryl, -CH₂-NR4R5, -SO₂NR4R5, und -C(O)NR4R5,
wobei der Substituent C₁-C₆-Alkyl mit C₁-C₆-Alkoxy, Fluor, Chlor, Trifluormethyl, Trifluormethoxy, Aryl, Heteroaryl, Heterocyclyl oder OH zumindest einfach substituiert sein kann,
und die Substituenten Aryl, Heteroaryl und Heterocyclyl mit C₁-C₆-Alkyl, C₁-C₆-Alkoxy, Fluor, Chlor, Trifluormethyl, Trifluormethoxy oder OH zumindest einfach substituiert sein können;
Aryl 5 bis 10-gliedriger, aromatischer Mono- oder Bicyclus;
Heteroaryl 5 bis 10-gliedriger, aromatischer, mono- oder bicyclischer Heterocyclus, der ein oder mehrere Heteroatome ausgewählt aus N, O und S enthält;
Heterocyclyl 5 bis 10-gliedriger, nicht-aromatischer, mono- oder bicyclischer Heterocyclus, der ein oder mehrere Heteroatome ausgewählt aus N, O und S enthält;
R4 und R5 unabhängig voneinander ausgewählt aus der Gruppe bestehend aus: Wasserstoff; unsubstituiertes oder zumindest monosubstituiertes C₁-C₁₀-Alkyl, C₂-C₆-Alkenyl, Phenyl, Indanyl, Heterocyclyl und Heteroaryl,
wobei die Substituenten ausgewählt sind aus der Gruppe bestehend aus: Phenyl, Heteroaryl, Heterocyclyl, -O-Phenyl, Fluor, -CN, -C(O)NH₂, -C(O)(C₁-C₃-Alkyl), -C(O)-Phenyl, -N(C₁-C₃-Alkyl)₂, -NH(C₁-C₃-Alkyl), -NH₂, -NH-Heteroaryl, -NH-C(O)-Heteroaryl, C₁-C₆-Alkyl, C₁-C₃-Alkoxy und Hydroxy,
und die Phenyl-, Heterocyclyl- und Heteroaryl-Fragmente dieser Substituenten können wiederum mit Fluor, Chlor, Brom, Oxo, -CF₃, -OCF₃, -NO₂, -CN, Phenyl, Pyridinyl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂, zumindest monosubstituiert sein; oder
R4 und R5 bilden zusammen mit dem Stickstoffatom, an das sie gebunden sind, unsubstituiertes oder zumindest monosubstituiertes Heterocyclyl,
wobei die Substituenten ausgewählt sind aus der Gruppe bestehend aus: Phenyl, Heteroaryl, Heterocyclyl, Oxo, Fluor, Chlor, -C(O)(C₁-C₃-Alkyl), -C(O)-Phenyl und Hydroxy, und die Phenyl-, Heterocyclyl- und Heteroaryl-Fragmente dieser Substituenten können wiederum mit Fluor oder C₁-C₃-Alkyl zumindest monosubstituiert sein;
und ihre physiologisch verträglichen Salze;
mit der Maßgabe, dass Ar nicht Triazinyl oder Chromanyl bedeutet,
und wenn X NH oder N(C₁-C₃-Alkyl) ist Ar nicht Pyridopyrazinyl oder Naphthyridinyl bedeutet.

2. Verbindung der Formel 1 gemäß Anspruch 1, worin bedeuten:
X O, NH oder N(C₁-C₃-Alkyl);
R1 Fluor, -OCF₃ oder C₁-C₄-Alkyl;
R2 Wasserstoff, Fluor, -OCF₃ oder C₁-C₄-Alkyl;
R3 Fluor, -OCF₃ oder C₁-C₄-Alkyl;
Ar unsubstituiertes oder einfach substituiertes Phenyl oder Heteroaryl, wobei die Substituenten ausgewählt sind aus der Gruppe bestehend aus:
Fluor, Chlor, -CF₃, -OCF₃, -CN, -C(O)(C₁-C₃-Alkyl), NH₂, -NHC(O)(C₁-C₃-Alkyl), Hydroxy, Oxo, C₁-C₃-Alkyl, C₁-C₃-Alkoxy, -NH(C₁-C₃-Alkyl), -N(C₁-C₃-Alkyl)₂, -SO₂(C₁-C₃-Alkyl), Heterocyclyl, Heteroaryl, Aryl, -O-Aryl, -O-Heteroaryl, -CH₂-NR4R5, -SO₂NR4R5, und -C(O)NR4R5,
wobei der Substituent C₁-C₃-Alkyl mit C₁-C₃-Alkoxy, Fluor, Chlor, Trifluormethyl, Trifluormethoxy, Aryl, Heteroaryl, Heterocyclyl oder OH zumindest einfach substituiert sein kann,
und die Substituenten Aryl, Heteroaryl und Heterocyclyl mit C₁-C₃-Alkyl, C₁-C₃-Alkoxy, Fluor, Chlor, Trifluormethyl, Trifluormethoxy oder OH zumindest einfach substituiert sein können;
R4 Wasserstoff oder C₁-C₃-Alkyl;
R5 ist ausgewählt aus der Gruppe bestehend aus; Wasserstoff; unsubstituiertes oder zumindest einfach substituiertes C₁-C₆-Alkyl, Cyclohexenyl, Indanyl, Phenyl, Pyrrolidinyl, Pyrrolyl, Pyrazolyl, Furanyl und Piperidinyl,
wobei die Substituenten ausgewählt sind aus der Gruppe bestehend aus: Fluor, -CN, -C(O)NH₂, -O-Phenyl, -C(O)-Phenyl, -N(CH₃)₂, C₁-C₃-Alkyl, C₁-C₃-Alkoxy, Hydroxy, unsubstituiertes oder zumindest monosubstituiertes Phenyl, Pyridinyl, Thienyl, Pyrimidinyl, Imidazolyl, Furanyl, Indolyl, Benzimidazolyl, Pyrazolyl, Morpholinyl, Pyrrolidinyl, 1,3-Benzodioxolyl, Piperidinyl, Tetrahydropyranyl, Triazolyl, Thiazolyl, Thiazolidinyl, Isoxazolyl und Dihydroisoxazolyl, deren Substituenten sind wiederum ausgewählt aus der Gruppe bestehend aus: Fluor, Chlor, Oxo, CF₃, -OCF₃, -NO₂, Phenyl, Pyridinyl, -NHC(O)CH₃, -COOH, Hydroxy, C₁-C₃-Alkyl, C₁-C₃-Alkoxy, -SO₂NH₂, -C(O)NH₂ und -N(CH₃)₂; oder
R4 und R5 bilden zusammen mit dem Stickstoffatom, an das sie gebunden sind, einen Rest ausgewählt aus der Gruppe bestehend aus: unsubstituiertes oder zumindest einfach substituiertes Piperidinyl, Pyrrolidinyl, Morpholinyl und Piperazinyl,
wobei die Substituenten ausgewählt sind aus der Gruppe bestehend aus: Fluor, -C(O)(C₁-C₃-Alkyl), Oxo, C₁-C₃-Alkyl, Hydroxy, unsubstituiertes oder zumindest einfach substituiertes Phenyl, Imidazolyl, Pyridinyl, Pyrimidinyl, Piperidinyl und Pyrrolidinyl, deren Substituenten sind wiederum Fluor oder C₁-C₃-Alkyl;
Aryl Phenyl, Indanyl oder Naphthyl;
Heteroaryl Pyridinyl, Thienyl, Pyrimidinyl, Imidazolyl, Furanyl, Indolyl, Benzimidazolyl, Pyrazolyl, 1,3-Benzodioxolyl, Triazolyl, Thiazolyl, Isoxazolyl, Pyrrolyl, Pyrazinyl, Oxazolyl, Pyridazinyl, Chinolinyl, Isochinolinyl, Benzofuranyl, 3-Oxo-1,3-dihydroisobenzofuranyl, 2,3-Dihydroindolyl oder 4,5,6,7-Tetrahydrobenzothiazolyl;
Heterocyclyl Morpholinyl, Pyrrolidinyl, Piperidinyl, Tetrahydropyranyl, Thiazolidinyl, Dihydroisoxazolyl, Piperazinyl oder Tetrahydrofuranyl;
und ihre physiologisch verträglichen Salze.

3. Verbindungen der Formel I gemäß Anspruch 1 und/oder 2, worin bedeuten:
X NH oder N(C₁-C₃-Alkyl);
R1 C₁-C₃-Alkyl;
R2 Wasserstoff;
R3 C₁-C₃-Alkyl;
Ar unsubstituiertes oder einfach substituiertes Phenyl, Indolyl, Benzofuranyl, Benzimidazolyl, Furanyl, Thienyl, Imidazolyl, Pyrimidinyl, Pyrazolyl, Isochinolinyl, Pyridinyl, Chinolinyl, oder 2,3-Dihydroindolyl,
wobei die Substituenten ausgewählt sind aus der Gruppe bestehend aus: Fluor, Chlor, -CF₃, -OCF₃, -CN, -C(O)(C₁-C₃-Alkyl), NH₂, -NHC(O)(C₁-C₃-Alkyl), Hydroxy, Oxo, C₁-C₃-Alkyl, C₁-C₃-Alkoxy, -NH(C₁-C₃-Alkyl), -N(C₁-C₃-Alkyl)₂, -SO₂(C₁-C₃-Alkyl), Heteroaryl, Phenyl, -O-Phenyl, -O-Heteroaryl, -CH₂-NR4R5, -SO₂NR4R5, und -C(O)NR4R5,
wobei der Substituent C₁-C₃-Alkyl mit C₁-C₃-Alkoxy, Fluor, Chlor, Trifluormethyl, Trifluormethoxy, Phenyl, Heteroaryl oder OH zumindest einfach substituiert sein kann,
und die Substituenten Phenyl und Heteroaryl mit C₁-C₃-Alkyl, C₁-C₃-Alkoxy, Fluor, Chlor, Trifluormethyl, Trifluormethoxy oder OH zumindest einfach substituiert sein können;
R4 Wasserstoff;
R5 ist ausgewählt aus der Gruppe bestehend aus
Wasserstoff; unsubstituiertes oder einfach substituiertes C₁-C₆-Alkyl, Cyclohexenyl, Indanyl, Phenyl, Pyrrolidinyl, Pyrrolyl, Pyrazolyl, Furanyl und Piperidinyl,
wobei die Substituenten ausgewählt sind aus der Gruppe bestehend aus: Fluor, -CN, -C(O)NH₂, -O-Phenyl, -C(O)-Phenyl, -N(CH₃)₂, C₁-C₃-Alkyl, C₁-C₃-Alkoxy, Hydroxy, unsubstituiertes oder zumindest monosubstituiertes Phenyl, Pyridinyl, Thienyl, Pyrimidinyl, Imidazolyl, Furanyl, Indolyl, Benzimidazolyl, Pyrazolyl, Morpholinyl, Pyrrolidinyl, 1,3-Benzodioxolyl, Piperidinyl, Tetrahydropyranyl, Triazolyl, Thiazolyl, Thiazolidinyl, Isoxazolyl und Dihydroisoxazolyl, deren Substituenten sind wiederum ausgewählt aus der Gruppe bestehend aus: Fluor, Chlor, Oxo, CF₃, -OCF₃, -NO₂, Phenyl, Pyridinyl, -NHC(O)CH₃, -COOH, Hydroxy, C₁-C₃-Alkyl, C₁-C₃-Alkoxy, -SO₂NH₂, -C(O)NH₂ und -N(CH₃)₂; oder
R4 und R5 bilden zusammen mit dem Stickstoffatom, an das sie gebunden sind, einen unsubstituierten oder zumindest einfach substituierten Pyrrolidinylrest,
wobei die Substituenten ausgewählt sind aus der Gruppe bestehend aus: C₁-C₃-Alkyl und Hydroxy;
Aryl Phenyl oder Indanyl;
Heteroaryl Pyridinyl, Chinolinyl, Indolyl, Benzofuranyl, Thienyl, Pyrimidinyl, Imidazolyl, Furanyl, Benzimidazolyl, Pyrazolyl, Thiazolyl, Isochinolinyl, Pyrrolyl oder 2,3-Dihydroindolyl;
und ihre physiologisch verträglichen Salze.
oder ein physiologisch verträgliches Salz davon;

4. Verbindung der Formel I gemäß einem oder mehreren der Ansprüche 1 bis 3, worin bedeuten:
X NH oder N-Methyl;
R1 Ethyl;
R2 Wasserstoff;
R3 Methyl;
Ar unsubstituiertes oder einfach substituiertes Phenyl, Indanyl, 5,6,7,8-Tetrahydronaphtyl, Pyridyl, Chinolinyl, oder 2,3-Dihydroindolyl,
wobei die Substituenten ausgewählt sind aus der Gruppe bestehend aus: -CN, -C(O)(C₁-C₃-Alkyl), -NHC(O)(C₁-C₃-Alkyl), Oxo, C₁-C₃-Alkyl, C₁-C₃-Alkoxy, -SO₂(C₁-C₃-Alkyl), Heteroaryl, -O-Heteroaryl, -CH₂-NR4R5, -SO₂NR4R5, und -C(O)NR4R5,
wobei der Substituent C₁-C₃-Alkyl mit Heteroaryl oder OH einfach substituiert sein kann,
und der Substituenten Heteroaryl mit C₁-C₃-Alkyl einfach substituiert sein kann;
R4 Wasserstoff
R5 Wasserstoff, C₁-C₃-Alkyl) oderPyridinyl;
R4 und R5 bilden zusammen mit dem Stickstoffatom, an das sie gebunden sind, einen Pyrrolidinylrest;
Heteroaryl Pyridinyl, Imidazolyl, oder Pyrimidinyl;
und ihre physiologisch verträglichen Salze.

5. Verbindungen der Formel I gemäß einem oder mehreren der Ansprüche 1 bis 4 ausgewählt aus der Gruppe bestehend aus:
3-(5-Ethyl-2-methyl-6-oxo-1,6-dihydro-pyridin-3-ylamino)-benzonitril;
3-(5-Ethyl-2-methyl-6-oxo-1,6-dihydro-pyridin-3-ylamino)-benzamid;
5-(3-Acetyl-phenylamino)-3-ethyl-6-methyl-1H-pyridin-2-on;
N-[3-(5-Ethyl-2-methyl-6-oxo-1,6-dihydro-pyridin-3-ylamino)-phenyl]-acetamid;
3-Ethyl-6-methyl-5-(chinolin-3-ylamino)-1H-pyridin-2-on;
4-(5-Ethyl-2-methyl-6-oxo-1,6-dihydro-pyridin-3-ylamino)-benzolsulfonamid;
3-Ethyl-6-methyl-5-[4-(pyridin-3-yloxy)-phenylamino]-1H-pyridin-2-on;
3-Ethyl-5-(4-imidazol-1-yl-phenylamino)-6-methyl-1H-pyridin-2-on;
5-(1-Acetyl-2,3-dihydro-1H-indol-6-ylamino)-3-ethyl-6-methyl-1H-pyridin-2- on;
4-(5-Ethyl-2-methyl-6-oxo-1,6-dihydro-pyridin-3-ylamino)-N-pyridin-2-yl-benzolsulfonamid;
3-Ethyl-6-methyl-5-(5-oxo-5,6,7,8-tetrahydro-naphthalin-2-ylamino)-1H-pyridin-2-on;
N-[6-(5-Ethyl-2-methyl-6-oxo-1,6-dihydro-pyridin-3-ylamino)-pyridin-3-yl]- acetamid;
6-(5-Ethyl-2-methyl-6-oxo-1,6-dihydro-pyridin-3-ylamino)-nicotinonitril;
3-Ethyl-6-methyl-5-(1-oxo-indan-5-ylamino)-1H-pyridin-2-on;
3-Ethyl-6-methyl-5-(pyridin-4-ylamino)-1H-pyridin-2-on;
3-Ethyl-6-methyl-5-(pyridin-2-ylamino)-1H-pyridin-2-on;
5-(2-Acetyl-phenylamino)-3-ethyl-6-methyl-1H-pyridin-2-on;
5-(4-Acetyl-phenylamino)-3-ethyl-6-methyl-1H-pyridin-2-on;
3-Ethyl-6-methyl-5-(pyridin-3-ylamino)-1H-pyridin-2-on;
3-Ethyl-5-(3-methoxy-phenoxy)-6-methyl-1H-pyridin-2-on;
3-(5-Ethyl-2-methyl-6-oxo-1,6-dihydro-pyridin-3-yloxy)-benzonitril;
3-Ethyl-6-methyl-5-(3-methylaminomethyl-phenylamino)-1H-pyridin-2-on;
3-Ethyl-5-(3-hydroxymethyl-phenylamino)-6-methyl-1H-pyridin-2-on;
3-Ethyl-5-(3-methansulfonyl-phenylamino)-6-methyl-1H-pyridin-2-on;
3- Ethyl-6-methyl-5-[3-(2-methyl-pyrimidin-4-yl)-phenylamino]-1H-pyridin-2-on;
3-Ethyl-6-methyl-5-(3-pyrrolidin-1-ylmethyl-phenylamino)-1H-pyridin-2-on;
3-Ethyl-5-[3-(1 -hydroxy-ethyl)-phenylamino]-6-methyl- H-pyridin-2-on;
3-Ethyl-6-methyl-5-(methyl-phenyl-amino)-1 H-pyridin-2-on;
sowie ihre physiologisch verträglichen Salze.

6. Verbindung der Formel I gemäß einem oder mehreren der Ansprüche 1 bis 5 und/oder ihre physiologisch verträglichen Salze zur Verwendung als Arzneimittel.

7. Verbindung der Formel I 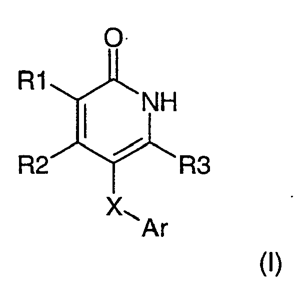 worin bedeuten:
R1 und R3 unabhängig voneinander Fluor, Methoxy, -OCF₃, C₂-C₃-Alkenyl oder C₁-C₄-Alkyl, das gegebenenfalls mit Chlor, Methoxy oder einem, zwei oder drei Fluoratomen substituiert ist;
R2 Wasserstoff, Fluor, Methoxy, -OCF₃, C₂-C₃-Alkenyl oder C₁-C₄-Alkyl, das gegebenenfalls mit Chlor, Methoxy oder einem, zwei oder drei Fluoratomen substituiert ist;
X O, S, NH oder N(C₁-C₃-Alkyl);
Ar unsubstituiertes oder zumindest einfach substituiertes Aryl oder Heteroaryl, wobei die Substituenten ausgewählt sind aus der Gruppe bestehend aus: Fluor, Chlor, Brom, -CF₃, -OCF₃, -NO₂, -CN, -C(O)(C₁-C₆-Alkyl), NH₂, -NHC(O)(C₁-C₆-Alkyl), Hydroxy, Oxo, C₁-C₆-Alkyl, C₁-C₆-Alkoxy, -NH(C₁-C₆-Alkyl), -N(C₁-C₆-Alkyl)₂, -SO₂(C₁-C₆-Alkyl), Heterocyclyl, Heteroaryl, Aryl, -O-Aryl, -O-Heteroaryl, -CH₂-NR4R5, -SO₂NR4R5, und -C(O)NR4R5,
wobei der Substituent C₁-C₆-Alkyl mit C₁-C₆-Alkoxy, Fluor, Chlor, Trifluormethyl, Trifluormethoxy, Aryl, Heteroaryl, Heterocyclyl oder OH zumindest einfach substituiert sein kann,
und die Substituenten Aryl, Heteroaryl und Heterocyclyl mit C₁-C₆-Alkyl, C₁-C₆-Alkoxy, Fluor, Chlor, Trifluormethyl, Trifluormethoxy oder OH zumindest einfach substituiert sein können;
Aryl 5 bis 10-gliedriger, aromatischer Mono- oder Bicyclus;
Heteroaryl 5 bis 10-gliedriger, aromatischer, mono- oder bicyclischer Heterocyclus, der ein oder mehrere Heteroatome ausgewählt aus N, O und S enthält;
Heterocyclyl 5 bis 10-gliedriger, nicht-aromatischer, mono- oder bicyclischer Heterocyclus, der ein oder mehrere Heteroatome ausgewählt aus N, O und S enthält;
R4 und R5 unabhängig voneinander ausgewählt aus der Gruppe bestehend aus: Wasserstoff; unsubstituiertes oder zumindest monosubstituiertes C₁-C₁₀-Alkyl, C₂-C₆-Alkenyl, Phenyl, Indanyl, Heterocyclyl und Heteroaryl,
wobei die Substituenten ausgewählt sind aus der Gruppe bestehend aus: Phenyl, Heteroaryl, Heterocyclyl, -O-Phenyl, Fluor, -CN, -C(O)NH₂, -C(O)(C₁-C₃-Alkyl), -C(O)-Phenyl, -N(C₁-C₃-Alkyl)₂, -NH(C₁-C₃-Alkyl), -NH₂, -NH-Heteroaryl, -NH-C(O)-Heteroaryl, C₁-C₆-Alkyl, C₁-C₃-Alkoxy und Hydroxy,
und die Phenyl-, Heterocyclyl- und Heteroaryl-Fragmente dieser Substituenten können wiederum mit Fluor, Chlor, Brom, Oxo, -CF₃, -OCF₃, -NO₂, -CN, Phenyl, Pyridinyl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂, zumindest monosubstituiert sein; oder
R4 und R5 bilden zusammen mit dem Stickstoffatom, an das sie gebunden sind, unsubstituiertes oder zumindest monosubstituiertes Heterocyclyl,
wobei die Substituenten ausgewählt sind aus der Gruppe bestehend aus: Phenyl, Heteroaryl, Heterocyclyl, Oxo, Fluor, Chlor, -C(O)(C₁-C₃-Alkyl), -C(O)-Phenyl und Hydroxy, und die Phenyl-, Heterocyclyl- und Heteroaryl-Fragmente dieser Substituenten können wiederum mit Fluor oder C₁-C₃-Alkyl zumindest monosubstituiert sein;
und /oder ihre physiologisch verträglichen Salze zur Hemmung der Poly(ADP-ribose)polymerase (PARP).

8. Verbindung der Formel I gemäß einem oder mehreren der Ansprüche 1 bis 5 und 7 und/oder ihre physiologisch verträglichen Salze zur Verwendung in Arzneimitteln zur Behandlung einer Krankheit, die ausgewählt ist aus der Gruppe bestehend aus: Gewebeschaden resultierend aus Zellschädigung oder Zelltod aufgrund von Nekrose oder Apoptose, neuronal vermittelter Gewebeschaden oder Erkrankungen, zerebrale Ischämie, Kopftrauma, Schlaganfall, Reperfusionsschaden, neurologische Störungen und neurodegenerative Erkrankungen, vaskulärer Schlaganfall, Herz-Kreislauf-Störungen, Myokardinfarkt, Myokardischämie, experimentelle allergische Enzephalomyelitis (EAE), Multiple Sklerose (MS), Ischämie in Bezug zu Herzchirurgie, altersbezogene Makulardegeneration, Arthritis, Arterosklerose, Krebs, degenerative Erkrankungen des Skelettmuskels mit folgender replikativer Seneszenz, Diabetes und diabetische Herzmuskelerkrankungen.

9. Verwendung von Verbindungen der Formel I gemäß einem oder mehreren der Ansprüche 1 bis 5 und 7 und/oder ihre physiologisch verträglichen Salze zur Herstellung eines Medikaments zur Behandlung einer Krankheit, die ausgewählt ist aus der Gruppe bestehend aus: Gewebeschaden resultierend aus Zellschädigung oder Zelltod aufgrund von Nekrose oder Apoptose, neuronal vermittelter Gewebeschaden oder Erkrankungen, zerebrale Ischämie, Kopftrauma, Schlaganfall, Reperfusionsschaden, neurologische Störungen und neurodegenerative Erkrankungen, vaskulärer Schlaganfall, Herz-Kreislauf-Störungen, Myokardinfarkt, Myokardischämie, experimentelle allergische Enzephalomyelitis (EAE), Multiple Sklerose (MS), Ischämie in Bezug zu Herzchirurgie, altersbezogene Makulardegeneration, Arthritis, Arterosklerose, Krebs, degenerative Erkrankungen des Skelettmuskels mit folgender replikativer Seneszenz, Diabetes und diabetische Herzmuskelerkrankungen.

10. Verwendung gemäß Anspruch 9, **dadurch gekennzeichnet, dass** die Krankheit ausgewählt ist aus der Gruppe bestehend aus: zerebrale Ischämie, Reperfusionsschaden, Herz-Kreislauf-Störungen, Myokardinfarkt, Myokardischämie und Ischämie in Bezug zu Herzchirurgie.

11. Verwendung gemäß Anspruch 9 oder 10, **dadurch gekennzeichnet, dass** die Krankheit Myokardinfarkt ist.

12. Pharmazeutische Zusammensetzung umfassend eine wirksame Menge von mindestens einer Verbindung oder einem physiologisch verträglichen Salz davon gemäß einem oder mehreren der Ansprüche 1 bis 5 und einen physiologisch verträglichen Träger.

## Claims

1. A compound of the formula I 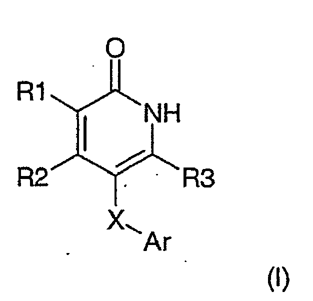 in which the meanings are:
R1 and R3 independently of one another fluorine, methoxy, -OCF₃, C₂-C₃-alkenyl or C₁-C₄-alkyl which is optionally substituted by chlorine, methoxy or one, two or three fluorine atoms;
R2 hydrogen, fluorine, methoxy, -OCF₃, C₂-C₃-alkenyl or C₁-C₄-alkyl which is optionally substituted by chlorine, methoxy or one, two or three fluorine atoms;
X O, S, NH or N (C₁-C₃-alkyl);
Ar unsubstituted or at least monosubstituted aryl or heteroaryl, where the substituents are selected from the group consisting of:
fluorine, chlorine, bromine, -CF₃, -OCF₃, -NO₂, -CN, -C(O)(C₁-C₆-alkyl), NH₂ , -NHC(O)(C₁-C₆-alkyl), hydroxy, oxo, C₁-C₆-alkyl, C₁-C₆-alkoxy, -NH(C₁-C₆-alkyl), -N(C₁-C₆-alkyl)₂, -SO₂(C₁-C₆-alkyl), heterocyclyl, heteroaryl, aryl, -O-aryl, -O-heteroaryl, -CH₂-NR4R5, -SO₂NR4R5, and -C(O)NR4R5,
where the C₁-C₆-alkyl substituent may be substituted at least once by C₁-C₆-alkoxy, fluorine, chlorine, trifluoromethyl, trifluoromethoxy, aryl, heteroaryl, heterycyclyl or OH,
and the aryl, heteroaryl and heterocyclyl substituents may be substituted at least once by C₁-C₆-alkyl, C₁-C₆-alkoxy, fluorine, chlorine, trifluoromethyl, trifluoromethoxy or OH;
aryl 5 to 10-membered aromatic mono- or bicycle;
heteroaryl 5 to 10-membered aromatic mono- or bicyclic heterocycle which comprises one or more heteroatoms selected from N, O and S;
heterocyclyl 5 to 10-membered nonaromatic mono- or bicyclic heterocycle which comprises one or more heteroatoms selected from N, O and S;
R4 and R5 independently of one another selected from the group consisting of:
hydrogen; unsubstituted or at least monosubstituted C₁-C₁₀-alkyl, C₂-C₆-alkenyl, phenyl, indanyl, heterocyclyl and heteroaryl,
where the substituents are selected from the group consisting of:
phenyl, heteroaryl, heterocyclyl, -O-phenyl, fluorine, -CN, -C(O)NH₂, -C(O)(C₁-C₃-alkyl), -C(O)-phenyl, -N(C₁-C₃-alkyl)₂, -NH(C₁-C₃-alkyl), -NH₂, -NH-heteroaryl, -NH-C(O)-heteroaryl, C₁-C₆-alkyl, C₁-C₃-alkoxy and hydroxy,
and the phenyl, heterocyclyl and heteroaryl fragments of these substituents may in turn be at least monosubstituted by fluorine, chlorine, bromine, oxo, -CF₃, -OCF₃, -NO₂, -CN, phenyl, pyridinyl, -NHC(O)(C₁-C₃-alkyl), -COOH, hydroxy, C₁-C₃-alkyl, C₁-C₃-alkoxy, -SO₂NH₂, -SO₂NH (C₁-C₃-alkyl), -SO₂N(C₁-C₃-alkyl)₂, -C(O)NH₂, -C(O)NH(C₁-C₃-alkyl), -C(O)N(C₁-C₃-alkyl)₂, -SO₂(C₁-C₃-alkyl), -NH₂, -NH (C₁-C₃-alkyl ) or -N (C₁-C₃-alkyl)₂; or
R4 and R5 form together with the nitrogen atom to which they are bonded unsubstituted or at least monosubstituted heterocyclyl,
where the substituents are selected from the group consisting of:
phenyl, heteroaryl, heterocyclyl, oxo, fluorine, chlorine, -C(O) (C₁-C₃-alkyl), -C(O)-phenyl and hydroxy,
and the phenyl, heterocyclyl and heteroaryl fragments of these substituents may in turn be at least monosubstituted by fluorine or C₁-C₃-alkyl;
and the physiologically tolerated salts thereof;
with the proviso that Ar is not triazinyl or chromanyl, and Ar is not pyridopyrazinyl or naphthyridinyl when X is NH or N(C₁-C₃-alkyl).

2. A compound of the formula I as claimed in claim 1,
in which the meanings are:
X O, NH or N(C₁-C₃-alkyl);
R¹ fluorine, -OCF₃ or C₁-C₄-alkyl;
R2 hydrogen, fluorine, -OCF₃ or C₁-C₄-alkyl;
R3 fluorine, -OCF₃ or C₁-C₄-alkyl;
Ar unsubstituted or monosubstituted phenyl or heteroaryl, where the substituents are selected from the group consisting of:
fluorine, chlorine, -CF₃, -OCF₃, -CN, -C(O)(C₁-C₃-alkyl), NH₂,
-NHC(O) (C₁-C₃-alkyl), hydroxy, oxo, C₁-C₃-alkyl , C₁-C₃-alkoxy, -NH(C₁-C₃-alkyl), -N(C₁-C₃-alkyl)₂, -SO₂(C₁-C₃-alkyl), heterocyclyl, heteroaryl, aryl, -O-aryl, -O-heteroaryl, -CH₂-NR4R5, -SO₂-NR4R5, and -C(O)NR4R5,
where the C₁-C₃-alkyl substituent may be at least monosubstituted by C₁-C₃-alkoxy, fluorine, chlorine, trifluoromethyl, trifluoromethoxy, aryl, heteroaryl, heterocyclyl or OH,
and the aryl, heteroaryl and heterocyclyl substituents may be at least monosubstituted by C₁-C₃-alkyl, C₁-C₃-alkoxy, fluorine, chlorine, trifluoromethyl, trifluoromethoxy or OH;
R4 hydrogen or C₁-C₃-alkyl ;
R5 is selected from the group consisting of; hydrogen; unsubstituted or at least monosubstituted C₁-C₆-alkyl, cyclohexenyl, indanyl, phenyl, pyrrolidinyl, pyrrolyl, pyrazolyl, furanyl and piperidinyl,
where the substituents are selected from the group consisting of: fluorine, -CN, -C(O)NH₂, -O-phenyl, -C(O)-phenyl, -N(CH₃)₂, C₁-C₃-alkyl , C₁-C₃-alkoxy, hydroxy, unsubstituted or at least monosubstituted phenyl, pyridinyl, thienyl, pyrimidinyl, imidazolyl, furanyl, indolyl, benzimidazolyl, pyrazolyl, morpholinyl, pyrrolidinyl, 1,3-benzodioxolyl, piperidinyl, tetrahydropyranyl, triazolyl, thiazolyl, thiazolidinyl, isoxazolyl
and dihydroisoxazolyl, whose substituents are in turn selected from the group consisting of: fluorine, chlorine, oxo, CF₃, -OCF₃, -NO₂, phenyl, pyridinyl, -NHC(O)CH₃, -COOH, hydroxy, C₁-C₃-alkyl, C₁-C₃-alkoxy, -SO₂NH₂, -C(O)NH₂ and -N(CH₃)₂; or
R4 and R5 form together with the nitrogen atom to which they are bonded a radical selected from the group consisting of: unsubstituted or at least monosubstituted piperidinyl, pyrrolidinyl, morpholinyl and piperazinyl,
where the substituents are selected from the group consisting of: fluorine, -C(O)(C₁-C₃-alkyl), oxo, C₁-C₃-alkyl, hydroxy, unsubstituted or at least monosubstituted phenyl, imidazolyl, pyridinyl, pyrimidinyl, piperidinyl and pyrrolidinyl, whose substituents are in turn fluorine or C₁-C₃-alkyl;
aryl phenyl, indanyl or naphthyl;
heteroaryl pyridinyl, thienyl, pyrimidinyl, imidazolyl, furanyl, indolyl, benzimidazolyl, pyrazolyl, 1,3-benzodioxolyl, triazolyl, thiazolyl, isoxazolyl, pyrrolyl, pyrazinyl, oxazolyl, pyridazinyl, quinolinyl, isoquinolinyl, benzofuranyl, 3-oxo-1,3-dihydroisobenzofuranyl, 2,3-dihydroindolyl or 4,5,6,7-tetrahydrobenzothiazolyl;
heterocyclyl morpholinyl, pyrrolidinyl, piperidinyl, tetrahydropyranyl, thiazolidinyl, dihydroisoxazolyl, piperazinyl or tetrahydrofuranyl;
and the physiologically tolerated salts thereof.

3. A compound of the formula I as claimed in claim 1 and/or 2, in which the meanings are:
X NH or N (C₁-C₃-alkyl);
R1 C₁-C₃-alkyl;
R2 hydrogen;
R3 C₁-C₃-alkyl ;
Ar unsubstituted or monosubstituted phenyl, indolyl, benzofuranyl, benzimidazolyl, furanyl, thienyl, imidazolyl, pyrimidinyl, pyrazolyl, isoquinolinyl, pyridinyl, quinolinyl, or 2,3-dihydroindolyl,
where the substituents are selected from the group consisting of:
fluorine, chlorine, -CF₃, -OCF₃, -CN, -C(O)(C₁-C₃-alkyl), NH₂,
-NHC (O) (C₁-C₃-alkyl), hydroxy, oxo, C₁-C₃-alkyl, C₁-C₃-alkoxy, -NH(C₁-C₃-alkyl), -N(C₁-C₃-alkyl)₂, -SO₂ (C₁-C₃-alkyl), heteroaryl, phenyl, -O-phenyl, -O-heteroaryl, -CH₂-NR4R5, -SO₂-NR4R5, and -C(O)NR4R5,
where the C₁-C₃-alkyl substituent may be at least monosubstituted by C₁-C₃-alkoxy, fluorine, chlorine, trifluoromethyl, trifluoromethoxy, phenyl, heteroaryl or OH,
and the phenyl and heteroaryl substituents may be at least monosubstituted by C₁-C₃-alkyl, C₁-C₃-alkoxy, fluorine, chlorine, trifluoromethyl, trifluoromethoxy or OH;
R4 hydrogen;
R5 is selected from the group consisting of hydrogen; unsubstituted or monosubstituted C₁-C₆-alkyl, cyclohexenyl, indanyl, phenyl, pyrrolidinyl, pyrrolyl, pyrazolyl, furanyl and piperidinyl,
where the substituents are selected from the group consisting of: fluorine, -CN, -C(O)NH₂, -O-phenyl, -C (O) -phenyl, -N(CH₃)₂, C₁-C₃-alkyl, C₁-C₃-alkoxy, hydroxy, unsubstituted or at least monosubstituted phenyl, pyridinyl, thienyl, pyrimidinyl, imidazolyl, furanyl, indolyl, benzimidazolyl, pyrazolyl, morpholinyl, pyrrolidinyl, 1,3-benzodioxolyl, piperidinyl, tetrahydropyranyl, triazolyl, thiazolyl, thiazolidinyl, isoxazolyl and dihydroisoxazolyl, whose substituents are in turn selected from the group consisting of: fluorine, chlorine, oxo, CF₃, -OCF₃, -NO₂, phenyl, pyridinyl, -NHC(O)CH₃, -COOH, hydroxy, C₁-C₃-alkyl , C₁-C₃-alkoxy, -SO₂NH₂, -C(O)NH₂ and -N(CH₃)₂; or
R4 and R5 form together with the nitrogen atom to which they are bonded an unsubstituted or at least monosubstituted pyrrolidinyl radical, where the substituents are selected from the group consisting of: C₁-C₃-alkyl and hydroxy;
aryl phenyl or indanyl;
heteroaryl pyridinyl, quinolinyl, indolyl, benzofuranyl, thienyl, pyrimidinyl, imidazolyl, furanyl, benzimidazolyl, pyrazolyl, thiazolyl, isoquinolinyl, pyrrolyl or 2,3-dihydroindolyl;
and the physiologically tolerated salts thereof.

4. A compound of the formula I as claimed in one or more of claims 1 to 3, in which the meanings are:
X NH or N-methyl;
R1 ethyl;
R2 hydrogen;
R3 methyl;
Ar unsubstituted or monosubstituted phenyl, indanyl, 5,6,7,8-tetrahydronaphthyl, pyridinyl, quinolinyl, or 2,3-dihydroindolyl,
where the substituents are selected from the group consisting of: -CN, -C(O)(C₁-C₃-alkyl), -NHC(O)(C₁-C₃-alkyl), oxo, C₁-C₃-alkyl, C₁-C₃-alkoxy, -SO₂ (C₁-C₃-alkyl), heteroaryl, -O-heteroaryl, -CH₂-NR4R5, -SO₂-NR4R5, and -C(O)NR4R5,
where the C₁-C₃-alkyl substituent may be monosubstituted by heteroaryl or OH,
and the heteroaryl substituent may be monosubstituted by C₁-C₃-alkyl;
R4 hydrogen
R5 hydrogen, C₁-C₃-alkyl) or pyridinyl;
R4 and R5 form together with the nitrogen atom to which they are bonded a pyrrolidinyl radical;
heteroaryl pyridinyl, imidazolyl, or pyrimidinyl;
and the physiologically tolerated salts thereof.

5. A compound of the formula I as claimed in one or more of claims 1 to 4 selected from the group consisting of:
3-(5-ethyl-2-methyl-6-oxo-1,6-dihydropyridin-3-ylamino)benzonitrile;
3-(5-ethyl-2-methyl-6-oxo-1,6-dihydropyridin-3-ylamino)benzamide;
5-(3-acetylphenylamino)-3-ethyl-6-methyl-1H-pyridin-2-one;
N-[3-(5-ethyl-2-methyl-6-oxo-1,6-dihydropyridin-3-ylamino)phenyl]acetamide;
3-ethyl-6-methyl-5-(quinolin-3-ylamino)-1H-pyridin-2-one;
4-(5-ethyl-2-methyl-6-oxo-1,6-dihydropyridin-3-ylamino)benzenesulfonamide;
3-ethyl-6-methyl-5-[4-(pyridin-3-yloxy)phenylamino]-1H-pyridin-2-one;
3-ethyl-5-(4-imidazol-1-ylphenylamino)-6-methyl-1H-pyridin-2-one;
5-(1-acetyl-2,3-dihydro-1H-indol-6-ylamino)-3-ethyl-6-methyl-1H-pyridin-2-one;
4-(5-ethyl-2-methyl-6-oxo-1,6-dihydropyridin-3-ylamino)-N-pyridin-2-ylbenzenesulfonamide;
3-ethyl-6-methyl-5-(5-oxo-5,6,7,8-tetrahydronaphthalen-2-ylamino)-1H-pyridin-2-one;
N-[6-(5-ethyl-2-methyl-6-oxo-1,6-dihydropyridin-3-ylamino)pyridin-3-yl]acetamide;
6-(5-ethyl-2-methyl-6-oxo-1,6-dihydropyridin-3-ylamino)nicotinonitrile;
3-ethyl-6-methyl-5-(1-oxoindan-5-ylamino)-1H-pyridin-2-one;
3-ethyl-6-methyl-5-(pyridin-4-ylamino)-1H-pyridin-2-one;
3-ethyl-6-methyl-5-(pyridin-2-ylamino)-1H-pyridin-2-one;
5-(2-acetylphenylamino)-3-ethyl-6-methyl-1H-pyridin-2-one ;
5-(4-acetylphenylamino)-3-ethyl-6-methyl-1H-pyridin-2-one;
3-ethyl-6-methyl-5-(pyridin-3-ylamino)-1H-pyridin-2-one;
3-ethyl-5-(3-methoxyphenoxy)-6-methyl-1H-pyridin-2-one;
3-(5-ethyl-2-methyl-6-oxo-1,6-dihydropyridin-3-yloxy)benzonitrile;
3-ethyl-6-methyl-5-(3-methylaminomethylphenylamino)-1H-pyridin-2-one;
3-ethyl-5-(3-hydroxymethylphenylamino)-6-methyl-1H-pyridin-2-one;
3-ethyl-5-(3-methanesulfonylphenylamino)-6-methyl-1H-pyridin-2-one;
3-ethyl-6-methyl-5-[3-(2-methylpyrimidin-4-yl)phenylamino]-1H-pyridin-2-one;
3-ethyl-6-methyl-5-(3-pyrrolidin-1-ylmethylphenylamino)-1H-pyridin-2-one;
3-ethyl-5-[3-(1-hydroxyethyl)phenylamino]-6-methyl-1H-pyridin-2-one;
3-ethyl-6-methyl-5-(methylphenylamino)-1H-pyridin-2-one;
and the physiologically tolerated salts thereof.

6. A compound of the formula I as claimed in one or more of claims 1 to 5 and/or its physiologically tolerated salts for use as pharmaceutical.

7. A compound of the formula I 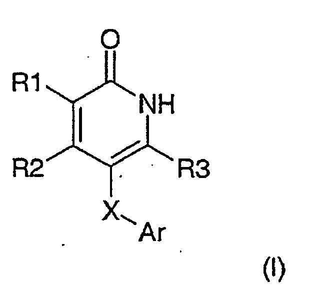 in which the meanings are:
R1 and R3 independently of one another fluorine, methoxy, -OCF₃, C₂-C₃-alkenyl or C₁-C₄-alkyl which is optionally substituted by chlorine, methoxy or one, two or three fluorine atoms;
R2 hydrogen, fluorine, methoxy, -OCF₃, C₂-C₃-alkenyl or C₁-C₄-alkyl which is optionally substituted by chlorine, methoxy or one, two or three fluorine atoms;
X O, S, NH or N(C₁-C₃-alkyl);
Ar unsubstituted or at least monosubstituted aryl or heteroaryl, where the substituents are selected from the group consisting of:
fluorine, chlorine, bromine, -CF₃, -OCF₃, -NO₂, -CN, -C(O)(C₁-C₆-alkyl), NH₂, -NHC(O)(C₁-C₆-alkyl), hydroxy, oxo, C₁-C₆-alkyl, C₁-C₆-alkoxy, -NH(C₁-C₆-alkyl), -N(C₁-C₆-alkyl)₂, -SO₂(C₁-C₆-alkyl), heterocyclyl, heteroaryl, aryl, -O-aryl, -O-heteroaryl, -CH₂-NR4R5, -SO₂NR4R5, and -C(O)NR4R5,
where the C₁-C₆-alkyl substituent may be substituted at least once by C₁-C₆-alkoxy, fluorine, chlorine, trifluoromethyl, trifluoromethoxy, aryl, heteroaryl, heterycyclyl or OH,
and the aryl, heteroaryl and heterocyclyl substituents may be substituted at least once by C₁-C₆-alkyl, C₁-C₆-alkoxy, fluorine, chlorine, trifluoromethyl, trifluoromethoxy or OH;
aryl 5 to 10-membered aromatic mono- or bicycle;
heteroaryl 5 to 10-membered aromatic mono- or bicyclic heterocycle which comprises one or more heteroatoms selected from N, O and S;
heterocyclyl 5 to 10-membered nonaromatic mono- or bicyclic heterocycle which comprises one or more heteroatoms selected from N, O and S;
R4 and R5 independently of one another selected from the group consisting of: hydrogen; unsubstituted or at least monosubstituted C₁-C₁₀-alkyl, C₂-C₆-alkenyl, phenyl, indanyl, heterocyclyl and heteroaryl,
where the substituents are selected from the group consisting of:
phenyl, heteroaryl, heterocyclyl, -O-phenyl, fluorine, -CN, -C(O)NH₂, -C(O)(C₁-C₃-alkyl), -C(O)-phenyl, -N(C₁-C₃-alkyl)₂, -NH(C₁-C₃-alkyl), -NH₂, -NH-heteroaryl, -NH-C(O)-heteroaryl, C₁-C₆-alkyl, C₁-C₃-alkoxy and hydroxy,
and the phenyl, heterocyclyl and heteroaryl fragments of these substituents may in turn be at least monosubstituted by fluorine, chlorine, bromine, oxo, -CF₃, -OCF₃, -NO₂, -CN, phenyl, pyridinyl, -NHC(O)(C₁-C₃-alkyl), -COOH, hydroxy, C₁-C₃-alkyl, C₁-C₃-alkoxy, -SO₂NH₂, -SO₂NH(C₁-C₃-alkyl), -SO₂N(C₁-C₃-alkyl)₂, -C(O)NH₂, -C(O)NH(C₁-C₃-alkyl), -C(O)N(C₁-C₃-alkyl)₂, -SO₂(C₁-C₃-alkyl), -NH₂, -NH(C₁-C₃-alkyl) or -N(C₁-C₃-alkyl)₂; or
R4 and R5 form together with the nitrogen atom to which they are bonded unsubstituted or at least monosubstituted heterocyclyl,
where the substituents are selected from the group consisting of:
phenyl, heteroaryl, heterocyclyl, oxo, fluorine, chlorine, -C(O)(C₁-C₃-alkyl), -C(O)-phenyl and hydroxy,
and the phenyl, heterocyclyl and heteroaryl fragments of these substituents may in turn be at least monosubstituted by fluorine or C₁-C₃-alkyl;
and/or its physiologically tolerated salts for inhibiting poly(ADP-ribose) polymerase (PARP).

8. A compound of the formula I as claimed in one or more of claims 1 to 5 and 7 and/or its physiologically tolerated salts for use in pharmaceuticals for the treatment of a disease selected from the group consisting of: tissue damage resulting from cell damage or cell death owing to necrosis or apoptosis, neuronally mediated tissue damage or disorders, cerebral ischemia, head trauma, stroke, reperfusion damage, neurological disturbances and neurodegenerative disorders, vascular stroke, cardiovascular disorders, myocardial infarction, mycocardial ischemia, experimental allergic encephalomyelitis (EAE), multiple sclerosis (MS), ischemia related to heart surgery, age-related macular degeneration, arthritis, arterosclerosis, cancer, degenerative skeletal muscle disorders with subsequent replicative senescence, diabetes and diabetic myocardial disorders.

9. The use of compounds of the formula I as claimed in one or more of claims 1 to 5 and 7 and/or their physiologically tolerated salts for the manufacture of a medicament for the treatment of a disease selected from the group consisting of: tissue damage resulting from cell damage or cell death owing to necrosis or apoptosis, neuronally mediated tissue damage or disorders, cerebral ischemia, head trauma, stroke, reperfusion damage, neurological disturbances and neurodegenerative disorders, vascular stroke, cardiovascular disorders, myocardial infarction, mycocardial ischemia, experimental allergic encephalomyelitis (EAE), multiple sclerosis (MS), ischemia related to heart surgery, age-related macular degeneration, arthritis, arterosclerosis, cancer, degenerative skeletal muscle disorders with subsequent replicative senescence, diabetes and diabetic myocardial disorders.

10. The use as claimed in claim 9, wherein the disease is selected from the group consisting of: cerebral ischemia, reperfusion damage, cardiovascular disorders, myocardial infarction, myocardial ischemia and ischemia related to heart surgery.

11. The use as claimed in claim 9 or 10, wherein the disease is myocardial infarction.

12. A pharmaceutical composition comprising an effective amount of at least one compound or of a physiologically tolerated salt thereof as claimed in one or more of claims 1 to 5 and a physiologically tolerated carrier.

## Revendications

1. Composés de formule I 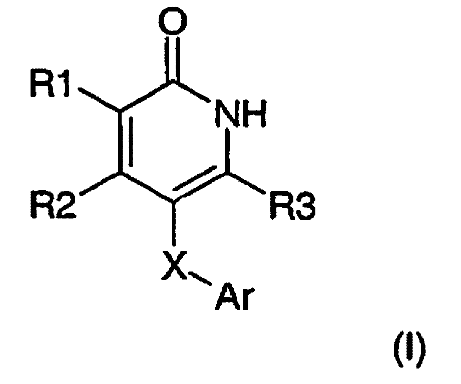 dans laquelle
R1 et R3 signifient, indépendamment l'un de l'autre fluor, méthoxy, -OCF₃, C₂-C₃-alcényle ou C₁-C₄-alkyle, qui est le cas échéant substitué par chlore, méthoxy ou un, deux ou trois atomes de fluor ;
R2 signifie hydrogène, fluor, méthoxy, -OCF₃, C₂-C₃-alcényle ou C₁-C₄-alkyle, qui est le cas échéant substitué par chlore, méthoxy ou un, deux ou trois atomes de fluor ;
X signifie O, S, NH ou N(C₁-C₃-alkyle) ;
Ar signifie aryle ou hétéroaryle non substitué ou au moins monosubstitué, les substituants étant choisis dans le groupe constitué par :
fluor, chlore, brome, -CF₃, -OCF₃, -NO₂, -CN, -C(O)(C₁-C₆-alkyle), NH₂, -NHC(O)(C₁-C₆-alkyle), hydroxy, oxo, C₁-C₆-alkyle, C₁-C₆-alcoxy, -NH(C₁-C₆-alkyle), -N(C₁-C₆-alkyle)₂, -SO₂ (C₁-C₆-alkyle) hétérocyclyle, hétéroaryle, aryle, -O-aryle, -O-hétéroaryle, -CH₂-NR4R5, -SO₂NR4R5, et -C(O)NR4R5, le substituant C₁-C₆-alkyle pouvant être au moins monosubstitué par C₁-C₆-alcoxy, fluor, chlore, trifluorométhyle, trifluorométhoxy, aryle, hétéroaryle, hétérocyclyle ou OH, et les substituants aryle, hétéroaryle et hétérocyclyle pouvant être au moins monosubstitués par C₁-C₆-alkyle, C₁-C₆-alcoxy, fluor, chlore, trifluorométhyle, trifluorométhoxy ou OH ;
aryle signifie un monocycle ou un bicycle aromatique de 5 à 10 chaînons ;
hétéroaryle signifie un hétérocycle monocyclique ou bicyclique aromatique, de 5 à 10 chaînons, qui contient un ou plusieurs hétéroatomes choisis parmi N, O et S ;
hétérocyclyle signifie un hétérocycle monocyclique ou bicyclique non aromatique, de 5 à 10 chaînons, qui contient un ou plusieurs hétéroatomes choisis parmi N, O et S ;
R4 et R5 sont choisis, indépendamment l'un de l'autre, dans le groupe constitué par : hydrogène ; C₁-C₁₀-alkyle, C₂-C₆-alcényle, phényle, indanyle, hétérocyclyle et hétéroaryle, non substitués ou au moins monosubstitués,
les substituants étant choisis dans le groupe constitué par: phényle, hétéroaryle, hétérocyclyle, -O-phényle, fluor, -CN, -C(O)NH₂, -C(O)(C₁-C₃-alkyle), -C(O)-phényle, -N(C₁-C₃-alkyle)₂, -NH(C₁-C₃-alkyle), -NH₂, -NH-hétéroaryle, -NH-C(O)-hétéroaryle, C₁-C₆-alkyle, C₁-C₃-alcoxy et hydroxy,
et les fragments phényle, hétérocyclyle et hétéroaryle de ces substituants peuvent à leur tour être au moins monosubstitués par fluor, chlore, brome, oxo, -CF₃, -OCF₃, -NO₂, -CN, phényle, pyridinyle, -NHC(O)(C₁-C₃-alkyle), -COOH, hydroxy, C₁-C₃-alkyle, C₁-C₃-alcoxy, -SO₂NH₂, -SO₂NH(C₁-C₃-alkyle), -SO₂N(C₁-C₃-alkyle)₂, -C(O)NH₂, -C (O) NH (C₁-C₃-alkyle) -C(O)N(C₁-C₃-alkyle)₂, -SO₂(C₁-C₃-alkyle), -NH₂, -NH(C₁-C₃-alkyle) ou -N(C₁-C₃-alkyle)₂ ; ou
R4 et R5 forment, ensemble avec l'atome d'azote, auquel ils sont liés, un hétérocyclyle non substitué ou au moins monosubstitué,
les substituants étant choisis dans le groupe constitué par : phényle, hétéroaryle, hétérocyclyle, oxo, fluor, chlore, -C(O)(C₁-C₃-alkyle), -C(O)-phényle et hydroxy,
et les fragments phényle, hétérocyclyle et hétéroaryle de ces substituants peuvent à nouveau être au moins monosubstitués par fluor ou C₁-C₃-alkyle ;
et leurs sels physiologiquement acceptables ;
à condition que Ar ne signifie pas triazinyle ni chromanyle,
et que, lorsque X représente NH ou N(C₁-C₃-alkyle), Ar ne signifie pas pyridopyrazinyle ni naphtyridinyle.

2. Composé de formule I selon la revendication 1, dans laquelle
X signifie O, NH ou N(C₁-C₃-alkyle) ;
R1 signifie fluor, -OCF₃ ou C₁-C₄-alkyle ;
R2 signifie hydrogène, fluor, -OCF₃ ou C₁-C₄-alkyle ;
R3 signifie fluor, -OCF₃ ou C₁-C₄-alkyle ;
Ar signifie phényle ou hétéroaryle non substitué ou monosubstitué, les substituants étant choisis dans le groupe constitué par :
fluor, chlore, -CF₃, -OCF₃, -CN, -C(O)(C₁-C₃-alkyle), NH₂, -NHC(O)(C₁-C₃-alkyle), hydroxy, oxo, C₁-C₃-alkyle, C₁-C₃-alcoxy, -NH(C₁-C₃-alkyle), -N(C₁-C₃-alkyle)₂, -SO₂(C₁-C₃-alkyle), hétérocyclyle, hétéroaryle, aryle, -O-aryle, -O-hétéroaryle, -CH₂-NR4R5, -SO₂NR4R5, et -C(O)NR4R5,
le substituant C₁-C₃-alkyle pouvant être au moins monosubstitué par C₁-C₃-alcoxy, fluor, chlore, trifluorométhyle, trifluorométhoxy, aryle, hétéroaryle, hétérocyclyle ou OH,
et les substituants aryle, hétéroaryle et hétérocyclyle pouvant être au moins monosubstitués par C₁-C₃-alkyle, C₁-C₃-alcoxy, fluor, chlore, trifluorométhyle, trifluorométhoxy ou OH ;
R4 représente hydrogène ou -C₁-C₃-alkyle ;
R5 est choisi dans le groupe constitué par : hydrogène ; C₁-C₆-alkyle, cyclohexényle, indanyle, phényle, pyrrolidinyle, pyrrolyle, pyrazolyle, furannyle et pipéridinyle, non substitués ou au moins monosubstitués,
les substituants étant choisis dans le groupe constitué par : fluor, -CN, -C(O)NH₂, -O-phényle, -C(O)-phényle, -N(CH₃)₂, C₁-C₃-alkyle, C₁-C₃-alcoxy, hydroxy, les radicaux phényle, pyridinyle, thiényle, pyrimidinyle, imidazolyle, furannyle, indolyle, benzimidazolyle, pyrazolyle, morpholinyle, pyrrolidinyle, 1,3-benzodioxolyle, pipéridinyle, tétrahydropyrannyle, triazolyle, thiazolyle, thiazolidinyle, isoxazolyle et dihydro-isoxazolyle, non substitués ou au moins monosubstitués, dont les substituants sont à nouveau choisis dans le groupe formé par: fluor, chlore, oxo, CF₃, -OCF₃, -NO₂, phényle, pyridinyle, -NHC (O) CH₃, -COOH, hydroxy, C₁-C₃-alkyle, C₁-C₃-alcoxy, -SO₂NH₂, -C(O)NH₂ et -N(CH₃)₂ ; ou
R4 et R5 forment ensemble avec l'atome d'azote auquel ils sont liés un radical choisi dans le groupe constitué par : pipéridinyle, pyrrolidinyle, morpholinyle et pipérazinyle, non substitués ou au moins monosubstitués,
les substituants étant choisis dans le groupe formé par : fluor, -C(O)(C₁-C₃-alkyle), oxo, C₁-C₃-alkyle, hydroxy, les radicaux phényle, imidazolyle, pyridinyle, pyrimidinyle, pipéridinyle et pyrrolidinyle, non substitués ou au moins monosubstitués, dont les substituants sont à nouveau fluor ou C₁-C₃-alkyle ;
aryle signifie phényle, indanyle ou naphtyle ;
hétéroaryle signifie pyridinyle, thiényle, pyrimidinyle, imidazolyle, furannyle, indolyle, benzimidazolyle, pyrazolyle, 1,3-benzodioxolyle, triazolyle, thiazolyle, isoxazolyle, pyrrolyle, pyrazinyle, oxazolyle, pyridazinyle, quinoléinyle, isoquinoléinyle, benzofurannyle, 3-oxo-1,3-dihydro-isobenzofurannyle, 2,3-dihydro-indolyle ou 4,5,6,7-tétrahydrobenzothiazolyle ;
hétérocyclyle signifie morpholinyle, pyrrolidinyle, pipéridinyle, tétrahydropyrannyle, thiazolidinyle, dihydro-isoxazolyle, pipérazinyle ou tétrahydrofurannyle ;
et ses sels physiologiquement acceptables.

3. Composés de formule I selon la revendication 1 et/ou 2, où
X signifie NH ou N(C₁-C₃-alkyle) ;
R1 signifie C₁-C₃-alkyle ;
R2 signifie hydrogène ;
R3 signifie C₁-C₃-alkyle ;
Ar signifie phényle, indolyle, benzofurannyle, benzimidazolyle, furannyle, thiényle, imidazolyle, pyrimidinyle, pyrazolyle, isoquinoléinyle, pyridinyle, quinoléinyle, ou 2,3-dihydro-indolyle, non substitués ou monosubstitués,
les substituants étant choisis dans le groupe constitué par : fluor, chlore, -CF₃, -OCF₃, -CN, -C(O)(C₁-C₃-alkyle), NH₂, -NHC(O)(C₁-C₃-alkyle), hydroxy, oxo, C₁-C₃-alkyle, C₁-C₃-alcoxy, -NH(C₁-C₃-alkyle), -N(C₁-C₃-alkyle)₂, -SO₂(C₁-C₃-alkyle), hétéroaryle, phényle, -O-phényle, -O-hétéroaryle, -CH₂-NR4R5, -SO₂NR4R5, et -C(O)NR4R5, où le substituant C₁-C₃-alkyle peut être au moins monosubstitué par C₁-C₃-alcoxy, fluor, chlore, trifluorométhyle, trifluorométhoxy, phényle, hétéroaryle ou OH,
et les substituants phényle et hétéroaryle peuvent être au moins monosubstitués par C₁-C₃-alkyle, C₁-C₃-alcoxy, fluor, chlore, trifluorométhyle, trifluorométhoxy ou OH;
R4 signifie hydrogène ;
R5 est choisi dans le groupe constitué par: hydrogène ; C₁-C₆-alkyle, cyclohexényle, indanyle, phényle, pyrrolidinyle, pyrrolyle, pyrazolyle, furannyle et pipéridinyle, non substitués ou monosubstitués,
les substituants étant choisis dans le groupe constitué par : fluor, -CN, -C(O)NH₂, -O-phényle, -C (O) -phényle, -N(CH₃)₂, C₁-C₃-alkyle, C₁-C₃-alcoxy, hydroxy, les radicaux phényle, pyridinyle, thiényle, pyrimidinyle, imidazolyle, furannyle, indolyle, benzimidazolyle, pyrazolyle, morpholinyle, pyrrolidinyle, 1,3-benzodioxolyle, pipéridinyle, tétrahydropyrannyle, triazolyle, thiazolyle, thiazolidinyle, isoxazolyle et dihydro-isoxazolyle, non substitués ou au moins monosubstitués, dont les substituants sont à nouveau choisis dans le groupe formé par : fluor, chlore, oxo, CF₃, -OCF₃, -NO₂, phényle, pyridinyle, -NHC(O)CH₃, -COOH, hydroxy, C₁-C₃-alkyle, C₁-C₃-alcoxy, -SO₂NH₂, -C (O) NH₂ et -N(CH₃)₂ ; ou
R4 et R5 forment ensemble avec l'atome d'azote auquel ils sont liés un radical pyrrolidinyle non substitué ou au moins monosubstitué,
les substituants étant choisis dans le groupe constitué par : C₁-C₃-alkyle et hydroxy ;
aryle signifie phényle ou indanyle ;
hétéroaryle signifie pyridinyle, quinoléinyle, indolyle, benzofurannyle, thiényle, pyrimidinyle, imidazolyle, furannyle, benzimidazolyle, pyrazolyle, thiazolyle, isoquinoléinyle, pyrrolyle ou 2,3-dihydro-indolyle ;
et leurs sels physiologiquement acceptables.

4. Composé de formule I selon l'une ou plusieurs des revendications 1 à 3, dans laquelle
X signifie NH ou N-méthyle ;
R1 signifie éthyle ;
R2 signifie hydrogène ;
R3 signifie méthyle ;
Ar signifie phényle, indanyle, 5,6,7,8-tétrahydronaphtyle, pyridinyle, quinoléinyle ou 2,3-dihydro-indolyle, non substitués ou monosubstitués, les substituants étant choisis dans le groupe constitué par: -CN, -C(O)(C₁-C₃-alkyle), -NHC(O)(C₁-C₃-alkyle), oxo, C₁-C₃-alkyle, C₁-C₃-alcoxy, -SO₂(C₁-C₃-alkyle), hétéroaryle, -O-hétéroaryle, -CH₂-NR4R5, -SO₂NR4R5, et -C(O)NR4R5,
le substituant C₁-C₃-alkyle pouvant être monosubstitué par hétéroaryle ou OH,
et le substituant hétéroaryle pouvant être monosubstitué par C₁-C₃-alkyle ;
R4 signifie hydrogène
R5 signifie hydrogène, C₁-C₃-alkyle ou pyridinyle ;
R4 et R5 forment ensemble avec l'atome d'azote auquel ils sont liés un radical pyrrolidinyle :
hétéroaryle signifie pyridinyle, imidazolyle, ou pyrimidinyle ;
et ses sels physiologiquement acceptables.

5. Composés de formule I selon l'une ou plusieurs des revendications 1 à 4, choisis dans le groupe constitué par :
3-(5-éthyl-2-méthyl-6-oxo-1,6-dihydropyridin-3-ylamino)-benzonitrile ;
3-(5-éthyl-2-méthyl-6-oxo-1,6-dihydropyridin-3-ylamino)-benzamide ;
5-(3-acétylphénylamino)-3-éthyl-6-méthyl-1H-pyridin-2-one ;
N-[3-(5-éthyl-2-méthyl-6-oxo-1,6-dihydropyridin-3-ylamino)-phényl]-acétamide ;
3-éthyl-6-méthyl-5-(quinoléin-3-ylamino)-1H-pyridin-2-one ;
4-(5-éthyl-2-méthyl-6-oxo-1,6-dihydropyridin-3-ylamino)-benzènesulfonamide ;
3-éthyl-6-méthyl-5[4-(pyridin-3-yloxy)-phénylamino]-1H-pyridin-2-one ;
3-éthyl-5-(4-imidazol-1-ylphénylamino)-6-méthyl-1H-pyridin-2-one ;
5-(1-acétyl-2,3-dihydro-1H-indol-6-ylamino)-3-éthyl-6-méthyl-1H-pyridin-2-one ;
4-(5-éthyl-2-méthyl-6-oxo-1,6-dihydropyridin-3-ylamino)-N-pyridin-2-yl-benzènesulfonamide ;
3-éthyl-6-méthyl-5-(5-oxo-5,6,7,8-tétrahydronaphtalén-2-ylamino)-1H-pyridin-2-one ;
N-[6-(5-éthyl-2-méthyl-6-oxo-1,6-dihydropyridin-3-ylamino)-pyridin-3-yl]-acétamide ;
6-(5-éthyl-2-méthyl-6-oxo-1,6-dihydropyridin-3-ylamino)-nicotinonitrile ;
3-éthyl-6-méthyl-5-(1-oxoindan-5-ylamino)-1H-pyridin-2-one ;
3-éthyl-6-méthyl-5-(pyridin-4-ylamino)-1H-pyridin-2-one ;
3-éthyl-6-méthyl-5-(pyridin-2-ylamino)-1H-pyridin-2-one ;
5-(2-acétylphénylamino)-3-éthyl-6-méthyl-1H-pyridin-2-one ;
5-(4-acétylphénylamino)-3-éthyl-6-méthyl-1H-pyridin-2-one ;
3-éthyl-6-méthyl-5-(pyridin-3-ylamino)-1H-pyridin-2-one ;
3-éthyl-5-(3-méthoxyphénoxy)-6-méthyl-1H-pyridin-2-one ;
3-(5-éthyl-2-méthyl-6-oxo-1,6-dihydropyridin-3-yloxy)-benzonitrile ;
3-éthyl-6-méthyl-5-(3-méthylaminométhylphénylamino)-1H-pyridin-2-one ;
3-éthyl-5-(3-hydroxyméthylphénylamino)-6-méthyl-1H-pyridin-2-one ;
3-éthyl-5-(3-méthanesulfonylphénylamino)-6-méthyl-1H-pyridin-2-one ;
3-éthyl-6-méthyl-5-[3-(2-méthylpyrimidin-4-yl)-phénylamino]-1H-pyridin-2-one ;
3-éthyl-6-méthyl-5-(3-pyrrolidin-1-ylméthylphénylamino)-1H-pyridin-2-one ;
3-éthyl-5-[3-(1-hydroxyéthyl)-phénylamino]-6-méthyl-1H-pyridin-2-one ;
3-éthyl-6-méthyl-5-(méthylphénylamino)-1H-pyridin-2-one ;
ainsi que leurs sels physiologiquement acceptables.

6. Composé de formule I selon l'une ou plusieurs des revendications 1 à 5 et/ou ses sels physiologiquement acceptables destiné à une utilisation comme médicament.

7. Composé de formule I 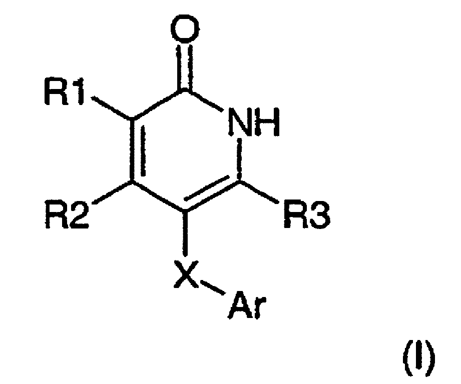 dans laquelle
R1 et R3 signifient, indépendamment l'un de l'autre fluor, méthoxy, -OCF₃, C₂-C₃-alcényle ou C₁-C₄-alkyle, qui est le cas échéant substitué par chlore, méthoxy ou un, deux ou trois atomes de fluor ;
R2 signifie hydrogène, fluor, méthoxy, -OCF₃, C₂-C₃-alcényle ou C₁-C₄-alkyle, qui est le cas échéant substitué par chlore, méthoxy ou un, deux ou trois atomes de fluor ;
X signifie O, S, NH ou N(C₁-C₃-alkyle) ;
Ar signifie aryle ou hétéroaryle non substitué ou au moins monosubstitué, les substituants étant choisis dans le groupe constitué par : fluor, chlore, brome, -CF₃, -OCF₃, -NO₂, -CN, -C(O)(C₁-C₆-alkyle),NH₂, -NHC(O)(C₁-C₆-alkyle), hydroxy, oxo, C₁-C₆-alkyle, C₁-C₆-alcoxy, -NH(C₁-C₆-alkyle), -N(C₁-C₆-alkyle)₂, -SO₂(C₁-C₆-alkyle), hétérocyclyle, hétéroaryle, aryle, -O-aryle, -O-hétéroaryle, -CH₂-NR4R5, -SO₂NR4R5, et -C(O)NR4R5,
le substituant C₁-C₆-alkyle pouvant être au moins monosubstitué par C₁-C₆-alCO_{XY}, fluor, chlore, trifluorométhyle, trifluorométhoxy, aryle, hétéroaryle, hétérocyclyle ou OH,
et les substituants aryle, hétéroaryle et hétérocyclyle pouvant être au moins monosubstitués par C₁-C₆-alkyle, C₁-C₆-alcoxy, fluor, chlore, trifluorométhyle, trifluorométhoxy ou OH ;
aryle signifie un monocycle ou un bicycle aromatique de 5 à 10 chaînons ;
hétéroaryle signifie un hétérocycle monocyclique ou bicyclique aromatique, de 5 à 10 chaînons, qui contient un ou plusieurs hétéroatomes choisis parmi N, O et S ;
hétérocyclyle signifie un hétérocycle monocyclique ou bicyclique non aromatique, de 5 à 10 chaînons, qui contient un ou plusieurs hétéroatomes choisis parmi N, O et S ;
R4 et R5 sont choisis, indépendamment l'un de l'autre, dans le groupe constitué par : hydrogène ; C₁-C₁₀-alkyle, C₂-C₆-alcényle, phényle, indanyle, hétérocyclyle et hétéroaryle, non substitués ou au moins monosubstitués,
les substituants étant choisis dans le groupe constitué par : phényle, hétéroaryle, hétérocyclyle, -O-phényle, fluor, -CN, -C(O)NH₂, -C(O)(C₁-C₃-alkyle), -C(O)-phényle, -N(C₁-C₃-alkyle)₂, -NH(C₁-C₃-alkyle), -NH₂, -NH-hétéroaryle, -NH-C(O)-hétéroaryle, C₁-C₆-alkyle, C₁-C₃-alcoxy et hydroxy,
et les fragments phényle, hétérocyclyle et hétéroaryle de ces substituants peuvent être à nouveau au moins monosubstitués par fluor, chlore, brome, oxo, -CF₃, -OCF₃, -NO₂, -CN, phényle, pyridinyle, -NHC (O) (C₁-C₃-alkyle), -COOH, hydroxy, C₁-C₃-alkyle, C₁-C₃-alcoxy, -SO₂NH₂, -SO₂NH(C₁-C₃-alkyle), -SO₂N(C₁-C₃-alkyle)₂, -C(O)NH₂, -C(O)NH(C₁-C₃-alkyle), -C(O)N(C₁-C₃-alkyle)₂, -SO₂(C₁-C₃-alkyle), -NH₂, -NH(C₁-C₃-alkyle) ou -N(C₁-C₃-alkyle)₂ ; ou
R4 et R5 forment ensemble avec l'atome d'azote auquel ils sont liés un hétérocyclyle non substitué ou au moins monosubstitué,
les substituants étant choisis dans le groupe constitué par: phényle, hétéroaryle, hétérocyclyle, oxo, fluor, chlore, -C(O)(C₁-C₃-alkyle), -C(O)-phényle et hydroxy,
et les fragments phényle, hétérocyclyle et hétéroaryle de ces substituants peuvent à nouveau être au moins monosubstitués par fluor ou C₁-C₃-alkyle ;
et/ou ses sels physiologiquement acceptables pour inhiber la poly(ADP-ribose)polymérase (PARP).

8. Composé de formule I selon l'une ou plusieurs des revendications 1 à 5 et 7 et/ou ses sels physiologiquement acceptables pour une utilisation dans des médicaments destinés au traitement d'une maladie, choisie dans le groupe constitué par: les dommages aux tissus provenant d'une dégradation cellulaire ou d'une mort cellulaire sur base d'une nécrose ou d'une apoptose, les dommages aux tissus ou les maladies d'origine neuronale, l'ischémie cérébrale, un trauma à la tête, une attaque, les dommages de reperfusion, les troubles neurologiques et les maladies de neurodégénérescence, une attaque vasculaire, les troubles cardiovasculaires, l'infarctus du myocarde, une ischémie du myocarde, une encéphalomyélite allergique expérimentale (EAE), la sclérose en plaques (multiple sclerosis - MS), une ischémie en rapport avec une chirurgie cardiaque, une dégénérescence maculaire due à la vieillesse, l'arthrite, l'artériosclérose, un cancer, les maladies de dégénérescence du muscle du squelette avec une sénescence de réplication consécutive, le diabète et les maladies diabétiques du muscle cardiaque.

9. Utilisation de composés formule I selon l'une ou plusieurs des revendications 1 à 5 et 7 et/ou leurs sels physiologiquement acceptables pour la préparation d'un médicament destiné au traitement d'une maladie, choisie dans le groupe constitué par : les dommages aux tissus provenant d'une dégradation cellulaire ou d'une mort cellulaire sur base d'une nécrose ou d'une apoptose, les dommages aux tissus ou les maladies d'origine neuronale, l'ischémie cérébrale, un trauma à la tête, une attaque, les dommages de reperfusion, les troubles neurologiques et les maladies de neurodégénérescence, une attaque vasculaire, les troubles cardiovasculaires, l'infarctus du myocarde, une ischémie du myocarde, une encéphalomyélite allergique expérimentale (EAE), la sclérose en plaques (multiple sclerosis - MS), une ischémie en rapport avec une chirurgie cardiaque, une dégénérescence maculaire due à la vieillesse, l'arthrite, l'artériosclérose, un cancer, les maladies de dégénérescence du muscle du squelette avec une sénescence de réplication consécutive, le diabète et les maladies diabétiques du muscle cardiaque.

10. Utilisation selon la revendication 9,
**caractérisée en ce que** la maladie est choisie dans le groupe constitué par : une ischémie cérébrale, les dommages de reperfusion, les troubles cardiovasculaires, l'infarctus du myocarde, une ischémie du myocarde et une ischémie en rapport avec une chirurgie cardiaque.

11. Utilisation selon la revendication 9 ou 10, **caractérisée en ce que** la maladie est l'infarctus du myocarde.

12. Préparation pharmaceutique, comprenant une quantité active d'au moins un composé ou d'un sel physiologiquement acceptable de celui-ci selon l'une ou plusieurs des revendications 1 à 5 et un support physiologiquement acceptable.
